# EUROPEAN PATENT APPLICATION

(11) **EP 4 268 895 A2**
(43) Date of publication of application: **01.11.2023**
(21) Application number: 23187468.6
(22) Date of filing: 16.07.2020
(51) Int. Cl.: A61P 27/02

(54) **USES OF CYP4V2 AND RDCVF IN PREPARATION OF DRUGS**

(30) Priority: 09.12.2019 CN 201911255192
(62) Divisional of application: 20898399.9
(71) Applicant: Chigenovo Co., Ltd., Beijing 102206 (CN)
(72) Inventor: YANG, Liping, Beijing, 100191 (CN); CHEN, Shaohong, Beijing, 102206 (CN); JIA, Ruixuan, Beijing, 100191 (CN); ZHANG, Tianfu, Beijing, 102206 (CN); ZHANG, Fan, Beijing, 102206 (CN); ZENG, Qiaoli, Beijing, 102206 (CN); HE, Saichao, Beijing, 102206 (CN); SHI, Tianyong, Beijing, 102206 (CN); HAO, Dandan, Beijing, 102206 (CN); JIANG, Shangwei, Beijing, 102206 (CN); PEI, Hongjie, Beijing, 102206 (CN); WANG, Xudong, Beijing, 102206 (CN); ZENG, Luying, Beijing, 102206 (CN)
(74) Representative: De Clercq & Partners

(57) **Abstract**

The present application relates to the use of CYP4V2 and RdCVF in the manufacture of a medicament for treating, alleviating, and/or preventing a disease or disorder associated with retinal pigment epithelium (RPE) atrophy. The present application also relates to an isolated nucleic acid molecule comprising a polynucleotide encoding CYP4V2 and a polynucleotide encoding RdCVF. The present application also relates to an amino acid sequence encoded by the isolated nucleic acid molecule, a vector comprising the isolated nucleic acid molecule, and a cell comprising the nucleic acid or the vector as well as use thereof in the manufacture of a medicament for treating, alleviating, and/or preventing a disease or disorder associated with retinal pigment epithelium (RPE) atrophy.

## Description

### TECHNICAL FIELD

The present application relates to the field of biomedicine, in particular to the use of CYP4V2 and RdCVF in the manufacture of a medicament.

### BACKGROUND

Bietti's crystalline dystrophy (BCD) is a rare disease of retinal degeneration, and the symptoms mainly include crystals (transparent coverings) in the cornea; small, yellow or white, crystalline deposits deposited in the photosensitive tissues of the retina; and progressive atrophy of the retina, choriocapillary, and choroid. The deposits may damage the retina, causing gradual loss of vision. Those having Bietti's crystalline dystrophy typically begin to perceive vision problems in their teens or twenties, and vision problems may be worsen at different rates in each eye. Even within the same family, the severity and progression of symptoms vary widely among different individuals. However, most patients become blind by the age of forties or fifties. It is estimated that 1 in 67,000 people worldwide has Bietti's crystalline dystrophy, and it is more common in East Asians, especially the Chinese and Japanese.

Current researches have shown that BCD is an autosomal recessive disease caused by CYP4V2 gene mutations. At present, the domestic and international researches of genes in BCD patients have found several gene mutation sites in CYP4V2. CYP4V2 gene is one of the proteins in cytochrome P450 superfamily, and the protein encoded by CYP4V2 gene is involved in the process of fatty acid metabolism. It is generally believed that in Bietti's crystalline dystrophy, the gene mutation of CYP4V2 destroys its enzymic function involved in fatty acid metabolism, thereby affecting the lipid decomposition. However, how CYP4V2 contributes to the specific signs and symptoms of BCD is still unclear. At present, the treatment of BCD mainly refers to the treatment of retinitis pigmentosa (RP). Although the study on CYP4V2 gene mutation provides the possibility for future gene therapy, there is no effective therapy at present.

### SUMMARY

The present application provides the use of CYP4V2 and RdCVF in the manufacture of a medicament for treating, alleviating, and/or preventing a disease or disorder associated with retinal pigment epithelium (RPE) atrophy.

In certain embodiments, the disease or disorder comprises Bietti's crystalline dystrophy (BCD).

In certain embodiments, the CYP4V2 is human CYP4V2.

In certain embodiments, the CYP4V2 comprises an amino acid sequence set forth in any of SEQ ID NOs: 76-82.

In certain embodiments, the polynucleotide encoding CYP4V2 comprises a nucleic acid sequence set forth in any of SEQ ID NOs: 62-68.

In certain embodiments, the RdCVF is human RdCVF.

In certain embodiments, the RdCVF comprises an amino acid sequence set forth in any of SEQ ID NOs: 83-89.

In certain embodiments, wherein the polynucleotide encoding RdCVF comprises a nucleic acid sequence set forth in any of SEQ ID NOs: 69-75.

In certain embodiments, the medicament comprises a polynucleotide encoding CYP4V2 and a polynucleotide encoding RdCVF.

In certain embodiments, the polynucleotide encoding CYP4V2 and the polynucleotide encoding RdCVF are located in different vectors.

In certain embodiments, the polynucleotide encoding CYP4V2 and the polynucleotide encoding RdCVF are located in a same vector.

In certain embodiments, the vector comprises a viral vector.

In certain embodiments, the vector is a viral vector, wherein the viral vector comprises an AAV vector.

In certain embodiments, the vector further comprises a promoter located at 5' end of the polynucleotide encoding CYP4V2 and operably linked to the polynucleotide encoding CYP4V2.

In certain embodiments, the vector further comprises a promoter located at 5' end of the polynucleotide encoding RdCVF and operably linked to the polynucleotide encoding RdCVF.

In certain embodiments, the promoter comprises a nucleotide sequence set forth in any of SEQ ID NOs: 1-12.

In certain embodiments, the vector further comprises a polyadenylation (PolyA) signal site located at 3' end of the polynucleotide encoding CYP4V2.

In certain embodiments, the vector further comprises a polyadenylation (PolyA) signal site located at 3' end of the polynucleotide encoding RdCVF.

In certain embodiments, the vector further comprises a polynucleotide encoding a self-cleaving peptide, located between the polynucleotide encoding CYP4V2 and the polynucleotide encoding RdCVF.

In certain embodiments, the self-cleaving peptide comprises P2A.

In certain embodiments, the polynucleotide encoding the self-cleaving peptide comprises a nucleotide sequence set forth in any of SEQ ID NOs: 22-25.

In certain embodiments, the vector sequentially comprises, in 5' to 3' direction: the promoter, the polynucleotide encoding CYP4V2, the polynucleotide encoding the self-cleaving peptide, the polynucleotide encoding RdCVF, and the PolyA signal site.

In certain embodiments, the vector sequentially comprises, in 5' to 3' direction: the promoter, the polynucleotide encoding CYP4V2, and the PolyA signal site; or alternatively, the vector sequentially comprises, in 5' to 3' direction: the promoter, the polynucleotide encoding RdCVF, and the PolyA signal site.

In certain embodiments, the vector further comprises an intron.

In certain embodiments, the intron comprises a nucleotide sequence set forth in any of SEQ ID NOs: 13-16.

In certain embodiments, the intron is located in the polynucleotide encoding CYP4V2, or located at 5' end of the polynucleotide encoding CYP4V2.

In certain embodiments, the intron is located in the polynucleotide encoding RdCVF, or located at 5' end of the polynucleotide encoding RdCVF.

In certain embodiments, the vector comprises a nucleotide sequence set forth in any of SEQ ID NOs: 90-116.

The present application also provides a vector combination for treating, alleviating, and/or preventing a disease or disorder associated with retinal pigment epithelium (RPE) atrophy, comprising a first vector and a second vector, wherein the first vector comprises a polynucleotide encoding CYP4V2, and the second vector comprises a polynucleotide encoding RdCVF.

In certain embodiments, the disease or disorder comprises Bietti's crystalline dystrophy (BCD).

In certain embodiments, the CYP4V2 is human CYP4V2.

In certain embodiments, the CYP4V2 comprises an amino acid sequence set forth in any of SEQ ID NOs: 76-82.

In certain embodiments, the polynucleotide encoding CYP4V2 comprises a nucleic acid sequence set forth in any of SEQ ID NOs: 62-68.

In certain embodiments, the RdCVF is human RdCVF.

In certain embodiments, the RdCVF comprises an amino acid sequence set forth in any of SEQ ID NOs: 83-89.

In certain embodiments, the polynucleotide encoding RdCVF comprises a nucleic acid sequence set forth in any of SEQ ID NOs: 69-75.

In certain embodiments, the vector comprises a viral vector.

In certain embodiments, the vector is a viral vector, wherein the viral vector comprises an AAV vector.

In certain embodiments, the first vector further comprises a promoter located at 5' end of the polynucleotide encoding CYP4V2 and operably linked to the polynucleotide encoding CYP4V2.

In certain embodiments, the second vector further comprises a promoter located at 5' end of the polynucleotide encoding RdCVF and operably linked to the polynucleotide encoding RdCVF.

In certain embodiments, the promoter comprises a nucleotide sequence set forth in any of SEQ ID NOs: 1-12.

In certain embodiments, the first vector further comprises a polyadenylation (PolyA) signal site located at 3' end of the polynucleotide encoding CYP4V2.

In certain embodiments, the second vector further comprises a polyadenylation (PolyA) signal site located at 3' end of the polynucleotide encoding RdCVF.

In certain embodiments, the first vector sequentially comprises, in 5' to 3' direction: the promoter, the polynucleotide encoding CYP4V2, and the PolyA signal site; and/or the second vector sequentially comprises, in 5' to 3' direction: the promoter, the polynucleotide encoding RdCVF, and the PolyA signal site.

In certain embodiments, the first vector and/or the second vector further comprise introns.

In certain embodiments, the intron comprises a nucleotide sequence set forth in any of SEQ ID NOs: 13-16.

In certain embodiments, the intron is located in the polynucleotide encoding CYP4V2, or located at 5' end of the polynucleotide encoding CYP4V2.

In certain embodiments, the intron is located in the polynucleotide encoding RdCVF, or located at 5' end of the polynucleotide encoding RdCVF.

In certain embodiments, the first vector comprises a nucleotide sequence set forth in any of SEQ ID NOs: 90-95; and/or the second vector comprises a nucleotide sequence set forth in any of SEQ ID NOs: 96-100.

The present application also provides one or more isolated nucleic acid molecules comprising a) a polynucleotide encoding CYP4V2, and b) a polynucleotide encoding RdCVF.

In certain embodiments, the CYP4V2 is human CYP4V2.

In certain embodiments, the CYP4V2 comprises an amino acid sequence set forth in any of SEQ ID NOs: 76-82.

In certain embodiments, the polynucleotide encoding CYP4V2 comprises a nucleic acid sequence set forth in any of SEQ ID NOs: 62-68.

In certain embodiments, the RdCVF is human RdCVF.

In certain embodiments, the RdCVF comprises an amino acid sequence set forth in any of SEQ ID NOs: 83-89.

In certain embodiments, the polynucleotide encoding RdCVF comprises a nucleic acid sequence set forth in any of SEQ ID NOs: 69-75.

In certain embodiments, the isolated nucleic acid molecule further comprises a promoter located at 5' end of the polynucleotide encoding CYP4V2 and operably linked to the polynucleotide encoding CYP4V2.

In certain embodiments, the isolated nucleic acid molecule further comprises a promoter located at 5' end of the polynucleotide encoding RdCVF and operably linked to the polynucleotide encoding RdCVF.

In certain embodiments, the promoter comprises a nucleotide sequence set forth in any of SEQ ID NOs: 1-12.

In certain embodiments, the isolated nucleic acid molecule comprises a polynucleotide encoding CYP4V2 and a polynucleotide encoding RdCVF.

In certain embodiments, the isolated nucleic acid molecule further comprises a polynucleotide encoding a self-cleaving peptide, located between the polynucleotide encoding CYP4V2 and the polynucleotide encoding RdCVF.

In certain embodiments, the self-cleaving peptide comprises P2A.

In certain embodiments, the polynucleotide encoding the self-cleaving peptide comprises a nucleotide sequence set forth in any of SEQ ID NOs: 22-25.

In certain embodiments, the isolated nucleic acid molecule further comprises a polyadenylation (PolyA) signal site located at 3' end of the polynucleotide encoding CYP4V2.

In certain embodiments, the isolated nucleic acid molecule further comprises a polyadenylation (PolyA) signal site located at 3' end of the polynucleotide encoding RdCVF.

In certain embodiments, the isolated nucleic acid molecule sequentially comprises, in 5' to 3' direction: the promoter, the polynucleotide encoding CYP4V2, the polynucleotide encoding the self-cleaving peptide, the polynucleotide encoding RdCVF, and the PolyA signal site.

In certain embodiments, the isolated nucleic acid molecule further comprises an intron.

In certain embodiments, the intron comprises a nucleotide sequence set forth in any of SEQ ID NOs: 13-16.

In certain embodiments, the intron is located in the polynucleotide encoding CYP4V2, or located at 5' end of the polynucleotide encoding CYP4V2.

In certain embodiments, the isolated nucleic acid molecule comprises a nucleotide sequence set forth in any of SEQ ID NOs: 101-115.

The present application also provides a vector comprising the isolated nucleic acid molecule described herein.

In certain embodiments, the vector is a viral vector.

In certain embodiments, the viral vector comprises an AAV vector.

The present application also provides a cell comprising the nucleic acid molecule described herein or the vector described herein.

The present application also provides a pharmaceutical composition comprising the isolated nucleic acid molecule described herein, the vector described herein, and/or the cell described herein.

The present application also provides the use of the nucleic acid molecule described herein, the vector described herein, or the cell described herein in the manufacture of a medicament for treating, alleviating, and/or preventing a disease or disorder associated with retinal pigment epithelium (RPE) atrophy.

In certain embodiments, the disease or disorder comprises Bietti's crystalline dystrophy.

Other aspects and advantages of the present application can be readily appreciated by those skilled in the art from the detailed descriptions below. Only exemplary embodiments of the present application are shown and described in the detailed descriptions below. As recognized by those skilled in the art, the contents of the present application enable those skilled in the art to make changes to the specific embodiments without departing from the spirit and scope of the invention disclosed in the present application. Accordingly, the accompanying drawings and the descriptions in the specification of the present application are only exemplary and not restrictive.

### DESCRIPTION OF THE DRAWINGS

The specific features of the invention disclosed in the present application are set forth in the appended claims. The characteristics and advantages of the invention disclosed in the present application can be better understood by reference to the exemplary embodiments described in detail below and the accompanying drawings. Brief descriptions of the accompanying drawings are as follows:
Fig. 1 shows the expression results of different CYP4V2 promoters in the present application.
Fig. 2 shows the expression results of the CAG, EF1a, OPEFS, and EFS promoters in the present application.
Figs. 3 and 4 show the expression enhancement effect of ligating an intron following the promoter in the present application.
Fig. 5 shows the effect of selecting different PolyA signal sites for the expression vector on the expression of the target gene in the present application.
Fig. 6 shows the expression of the expression vector containing CYP4V2 and RdCVF genes in 293T cells in the present application.
Fig. 7 shows the expression of the expression vector containing CYP4V2 and RdCVF genes in ARPE-19 cells in the present application.
Fig. 8 shows the infection of mouse retina by different serotypes of AAV viruses in the present application, wherein the double arrows indicate the expression range of the EGFP reporter gene.
Fig. 9 shows the morphology of renal epithelial cells, IPS cells, and RPE cells observed by fluorescence microscopy during the preparation of RPE cells in the present application.
Fig. 10A shows the effect of the virus titer on the death of human iPSC-differentiated RPE cells in the present application, Figs. 10B and 10C show the effect of the virus titer on the expressions of inflammatory factors NLRP3 and TNF-α in human iPSC-differentiated RPE cells, respectively, and Fig. 10D shows the expression of the target gene in human iPSC-differentiated RPE cells under different virus titers.
Fig. 11 shows the expression of CYP4V2 and RdCVF after the infections of human iPSC-differentiated RPE cells by various viruses in the present application.
Fig. 12 shows the effects of the treatments with viruses comprising different promoters and CYP4V2 gene in BCD mice on the fundus crystalline deposition in the present application.
Fig. 13 shows the statistical result for the effects of the treatments with viruses comprising different promoters and CYP4V2 gene in BCD mice on the fundus crystalline deposition in the present application.
Fig. 14 shows the results of CYP4V2 immunofluorescence staining after the treatments with viruses comprising different promoters and CYP4V2 gene in BCD mice in the present application.
Fig. 15 shows the effects of the treatments with different doses of viruses comprising CYP4V2 gene in BCD mice on the fundus crystalline deposition in the present application.
Fig. 16 shows the statistical result for the effects of the treatments with different doses of viruses comprising CYP4V2 gene in BCD mice on the fundus crystalline deposition in the present application.
Fig. 17 shows the effects of the treatments with different doses of viruses comprising CYP4V2 gene in BCD mice on the expression levels of inflammatory factors TNF-α, IFN-γ, and NLRP3 in the present application.
Fig. 18 shows the effects of the treatments with various viruses in BCD mice on the fundus crystalline deposition in the present application.
Fig. 19 shows the statistical result for the effects of the treatments with various viruses in BCD mice on the fundus crystalline deposition in the present application.
Fig. 20 shows the effects of the treatments with various viruses in BCD mice on the retinal function (electroretinogram (ERG)) in the present application.
Fig. 21 shows the results of CYP4V2 and RdCVF immunofluorescence staining after the treatments with various viruses in BCD mice in the present application.
Fig. 22 shows the effects of the treatments with various viruses in BCD mice on the number and morphology of RPE cells in the present application.
Fig. 23 shows the statistical result for the effects of the treatments with various viruses in BCD mice on the number of RPE cells in the present application.
Fig. 24 shows the effects of the treatments with various viruses that do not contain an intron following the promoter in BCD mice on the retinal function (electroretinogram (ERG)) in the present application.
Fig. 25 shows the effects of the treatments with various viruses containing the p1 promoter in BCD mice on the retinal function (electroretinogram (ERG)) in the present application.
Fig. 26 shows the effects of the treatments with various viruses containing the WPRE-SV40 poly A signal site in BCD mice on the retinal function (electroretinogram (ERG)) in the present application.
Fig. 27 shows the effects of the treatments with various viruses that do not contain the signal site in BCD mice on the retinal function (electroretinogram (ERG)) in the present application.

### DESCRIPTION OF EMBODIMENTS

The embodiments of the invention in the present application are described below by certain specific examples, and those skilled in the art can easily understand other advantages and effects of the invention in the present application from the contents disclosed in this specification.

The present application is further described below: in the present invention, unless otherwise specified, the scientific and technical terms used herein have the meanings commonly understood by those skilled in the art. Moreover, the related terms and laboratory procedures in protein and nucleic acid chemistry, molecular biology, cell and tissue culture, microbiology, and immunology as used herein are the terms and routine procedures widely used in the corresponding fields. Meanwhile, for a better understanding of the present invention, the definitions and explanations of related terms are provided below.

As used herein, the term "isolated" generally refers to being obtained from the natural state by an artificial means. If an "isolated" substance or ingredient occurs in nature, it may be due to a change in its natural environment, or the separation of this substance from the natural environment, or both. For example, a certain non-isolated polynucleotide or polypeptide is naturally existed in a living animal body, and the same polynucleotide or polypeptide with high purity isolated from this natural state is called "isolated." The term "isolated" neither excludes the admixture of artificial or synthetic substances, nor excludes the presence of other impure substances that do not affect the activity of the substance.

As used herein, the term "isolated nucleic acid molecule" generally refers to an isolated form of nucleotides, deoxyribonucleotides, or ribonucleotides of any length, or analogs isolated from their natural environments or artificially synthesized.

As used herein, the term "CYP4V2" generally refers to a protein that is member 2 of subfamily V of cytochrome P450 family 4. Cytochrome P450, also known as CYP450, usually refers to a family of ferroheme proteins, belonging to a class of monooxygenases, and involved in the metabolism of endogenous substances or exogenous substances including drugs and environmental compounds. According to the homology degree of amino acid sequence, the members are divided into three levels: family, subfamily, and individual enzymes. The cytochrome P450 enzyme system may be abbreviated as CYP, wherein the family is represented by Arabic number, the subfamily is represented by English capital letter, and the individual enzyme is represented by Arabic number, such as CYP4V2 herein. The human CYP4V2 gene (HGNC: 23198) has a full length of 19.28kb, located at 4q35, has 11 exons, and plays an important role in fatty acid metabolism (Kumar S., Bioinformation, 2011, 7:360-365). CYP4V2 is expressed almost in all tissues, but is expressed at a higher level in the retina and retinal pigment epithelium while at a slightly lower level in the cornea tissues, and the mutations in the CYP4V2 gene may result in BCD (Li et al., Am J Hum Genet. 74:817-826, 2004).

As used herein, the term "RdCVF", also known as Rod derived cone survival factor, generally refers to a truncated thioredoxin-like protein that lacks enzymatic activity for thiol redox. RdCVF is a splicing variant of the nucleoredoxin-like 1 (*Nxnl1*) gene. Another splicing product of this gene is RdCVFL, an active thioredoxin that protects its binding ligand (i.e., a microtubule-associated protein TAU) from oxidation and aggregation (Elachouri *et al*., 2015; and Fridlich *et al*., 2009). Mice deficient in the *Nxnl1* gene can exhibit age-dependent losses of rod and cone functions and cone degeneration, as well as rod and cone hypersensitivity to oxidative stress (Cronin *et al*., 2010). The expression of *Nxnl1* can be rod-dependent, which is significantly reduced after rod death in retinitis pigmentosa (RP) (Delyfer *et al*., 2011; Reichman *et al*., 2010). RdCVF can protect the cone function in several different genotypes of retinitis pigmentosa (RP) models (Byrne *et al*., 2015; Le' veillard *et al*., 2004; Yang *et al*., 2009). RdCVF molecules can be secreted out of cells as signal peptides to promote the glucose absorption of photoreceptor cells, which is beneficial for maintaining the survival of photoreceptor cells. The *Nxnl1* gene is conserved in human, chimpanzee, rhesus monkey, dog, cow, rat, mouse, chicken, zebrafish, and frog. Also known as *Txnl6* in human, *Nxnl1* is located at 19p13.11 and comprises two exons. *Nxnl1* can be ubiquitously expressed in human tissues, including lens, retina, stomach, kidney, heart, colon, and spleen, with relatively higher expression levels in lens and retina.

As used herein, the term "promoter" generally refers to a deoxyribonucleic acid (DNA) sequence that enables the transcription of a particular gene. The promoter can be recognized by RNA polymerase, and initiate the transcription and synthesis of RNA. During the synthesis of ribonucleic acid (RNA), the promoter can interact with the transcription factor for regulating the gene transcription, to control the initiation time and expression degree of the gene expression (transcription). The promoter comprises the core promoter region and the regulatory region, and is located in the regulatory sequence that controls the gene expression and upstream of the gene transcription initiation site (5' direction of the DNA antisense strand), and itself has no compilation function. According to the mode of action and function, the promoter is divided into three categories: constitutive promoter (consistent activity in most or all tissues), specific promoter (tissue specificity or specific for developmental stage), and inducible promoter (regulated by external chemical or physical signal).

As used herein, the term "operably linked" generally refers to placing the regulatory sequence necessary for the expression of a coding sequence at an appropriate position relative to the coding sequence so as to effect the expression of the coding sequence. For example, when a first nucleic acid sequence is in a functional relationship with a second nucleic acid sequence, the first nucleic acid sequence is operably linked to the second nucleic acid sequence. In certain embodiments, the arrangement of coding sequences and transcription control elements in an expression vector can be represented. The control element may include promoter, enhancer, and termination element. For example, if a promoter influences the transcription or expression of a coding sequence, the promoter is operably linked to the coding sequence. In certain embodiments, "operably linked" can also refer to the ligation of a target gene into a vector such that transcription and translation control sequences within the vector exert their intended functions of regulating the transcription and translation of the target gene.

As used herein, the term "self-cleaving peptide", also known as 2A peptide (2A self-cleaving peptide), generally refers to a class of peptide fragments having 18-22 amino acid residues in length, which can induce the intracellular self-cleavages of recombinant proteins containing 2A peptides. The 2A peptide is generally derived from the 2A region of the viral genome. In genetic engineering operations, the 2A peptide can divide a peptide chain translated from an open reading frame (ORF) into several independent peptide chains. In certain embodiments, if two proteins need to be expressed separately (for example, one protein needs to enter the nucleus and the other protein needs to be expressed in the cytoplasm), and it is also desired to construct only one open reading frame in the vector, then it can be achieved by inserting a segment of 2A peptide sequence into their coding regions. In certain embodiments, if a fusion protein from the fusion of two proteins has no function, a sequence encoding a 2A peptide can be inserted between the coding regions for these two proteins, or the linking peptide can be replaced with a 2A peptide, so that after the translation is completed, these two proteins are separated from each other, and folded independently, thus providing the possibility to restore the functions of these two proteins. The 2A peptide may include P2A, E2A, F2A, and T2A, all named by the virus of origin. Among them, P2A is derived from the 2A peptide of Porcine teschovirus.

As used herein, the term "polyadenylation (PolyA) sequence", also known as polyadenylation tail and PolyA tail, generally refers to a stretch of tens to hundreds of single adenosines added at the 3' end of mRNA after transcription. The polyadenylation usually occurs during and after the transcription of deoxyribonucleic acid (DNA) into ribonucleic acid (RNA) in the nucleus, and this reaction is usually completed by PolyA polymerase. In the eukaryote, the polyadenylation is a mechanism by which the mRNA molecule is interrupted at its 3' end, and the PolyA sequence can protect mRNA from the attack of exonuclease, and is very important for the nuclear export, translation and stability of mRNA.

As used herein, the term "polyadenylation (PolyA) signal site" generally refers to a base sequence located at the 3' end of messenger RNA (mRNA) that can be recognized by the polyadenylation-related cleavage factor. Usually, it is also a cis-regulatory signal on the mRNA. In general, the process of tailing (i.e., polyadenylation) begins after the termination of transcription, and tens to hundreds of single adenosines are added following 3' UTR in mRNA by the polyadenylation-related cleavage factor under the regulation of the PolyA signal site. The common tailing signals include SV40, BGH, HSV, TK signals, and the like. The polyadenylation-related cleavage factors may include cleavage/polyadenylation specific factor (CPSF), cleavage stimulation factor (CstF), cleavage factor I (CFI), cleavage factor II (CFII). The PolyA signal site may usually comprise an AAUAAA sequence, but it varies among eukaryotic groups. For example, most human PolyA signal sites comprise an AAUAAA sequence, but this sequence is less common in plants and fungi.

As used herein, the term "intron" may include a DNA fragment that is transcribed but removed from an RNA transcript by splicing together either end of a (exon) sequence. An intron is generally considered to be an interfering sequence within the protein coding region of a gene, and generally do not contain the information represented by the protein produced by the gene.

As used herein, the term "vector" generally refers to a nucleic acid delivery vehicle into which a polynucleotide encoding a protein can be inserted to express the protein. Through the transformation, transduction, or transfection of a host cell by the vector, the genetic elements carried by the vector are expressed in the host cell. For example, the vector comprises: plasmid; phagemid; cosmid; artificial chromosome, such as yeast artificial chromosome (YAC), bacterial artificial chromosome (BAC), or P1-derived artificial chromosome (PAC); phage, such as λ phage or M13 phage; viral vector; and the like. A vector may contain a variety of elements controlling expressions, including promoter sequence, transcription initiation sequence, enhancer sequence, selection element, and reporter gene. Additionally, the vector may also contain a replication origin. The vector may also comprise a component contributing to the entry into a cell, such as viral particle, liposome, or protein coat, but not limited to these substances.

As used herein, the term "viral vector" generally refers to a non-wild-type recombinant viral particle serving as a gene delivery vehicle and containing a recombinant viral genome packaged inside a viral capsid. The animal virus species used as the vector may include retrovirus (including lentivirus), adenovirus, adeno-associated virus (AAV), herpesvirus (such as herpes simplex virus), poxvirus, baculovirus, papillomavirus, and papovavirus (such as SV40).

As used herein, the term "AAV vector," also known as adeno-associated viral vector, generally refers to adenovirus itself or derivatives thereof. The adeno-associated virus (AAV) generally refers to a class of single-stranded DNA viruses belonging to the Dependovirus genus in the Parvoviridae family. The AAV genome can comprise inverted terminal repeats (ITRs) at both ends of the DNA strand, and two open reading frames (ORFs). The open reading frame may include *rep* and *cap. Rep* consists of multiple overlapping genes encoding Rep proteins required for the AAV life cycle, and *cap* comprises overlapping nucleotide sequences encoding capsid proteins, wherein the nucleotide sequences may include VP1, VP2, and VP3. The capsid proteins interact to form the capsid. In the absence of a helper virus, AAV can integrate its genome into a specific locus (AAVS locus) on the human chromosome 19, until a helper virus rescues it from latency (Kotin et al., 1990). It is generally believed that AAV is predominantly in the non-integration form. The site-specific integration capability, natural deficiency, and low immunogenicity of AAV make it an ideal gene therapy vector. AAV has a variety of common serotypes, and more than 100 virus variants. In the present application, the AAV capsid, ITR, and other selected AAV components are selected from any AAV, including but not limited to, AAV1, AAV2, AAV3, AAV4, AAV5, AAV6, AAV7, AAV8, AAV9, AAV8bp, AAV7M8, and AAVAnc80, variants of any known or mentioned AAV, or variants or mixtures thereof.

As used herein, the term "cell" can generally be or has been a single cell, cell line or cell culture of a recipient for the nucleic acid molecule or vector. The cell may comprise the nucleic acid molecule described herein or the vector described herein. The cell may include the progeny of a single cell. Due to the natural, accidental, or intentional mutation, the progeny may not necessarily be completely identical to the original parent cell (either morphologically in total DNA complement, or genomically). The cell may include a cell transfected *in vitro* with the vector described herein. The cell may be bacterial cell (e.g., *E. coli*)*,* yeast cell, or other eukaryotic cells, such as COS cell, Chinese hamster ovary (CHO) cell, HeLa cell, HEK293 cell, COS-1 cell, NS0 cell or myeloma cell. In certain embodiments, the cell is a mammalian cell. In certain embodiments, the mammalian cell is HEK293T cell.

As used herein, the term "pharmaceutical composition" generally refers to a composition suitable for administration to a patient such as human patient. For example, the pharmaceutical composition described herein may comprise the nucleic acid molecule described herein, the vector described herein, and/or the cell described herein, and optionally a pharmaceutically acceptable adjuvant. In addition, the pharmaceutical composition may also comprise one or more (pharmaceutically effective) vehicles, stabilizers, excipients, diluents, solubilizers, surfactants, emulsifiers, and/or preservatives for suitable formulations. The acceptable ingredients of the composition are not toxic to recipients at the dosages and concentrations employed. The pharmaceutical composition of the invention includes, but is not limited to, liquid, frozen, and lyophilized compositions.

As used herein, the term "preventing" generally refers to the prophylactic administration of a combination to a healthy subject to prevent the occurrence of a certain disease or disorder. It may also include the prophylactic administration of the combination to a patient in the early stage of an allergic disease to be treated. The term "preventing" does not require 100% elimination of the likelihood of a disease or disorder; in other words, the term "preventing" generally means that the likelihood of a disease or disorder is reduced in the presence of the administrated combination.

As used herein, the term "alleviating" refers to reducing, diminishing, or retarding a certain condition, disease, disorder, or phenotype. The condition, disease, disorder, or phenotype may include subjective perceptions of the subject such as pain, dizziness, or other physiological disturbances, or focus conditions detected by medical laboratory means.

As used herein, the term "treating" generally refers to a clinical intervention for altering the natural course of the treated individual or cell in a clinical pathological process. It may include improving the disease status, eliminating lesions, or improving the prognosis.

As used herein, the term "retinal pigment epithelium (RPE)" generally refers to a layer of pigment cells immediately outside the retinal sensory nerves. The retinal pigment epithelium consists of a single layer of hexagonal cells that contain dense pigment granules. The retinal pigment epithelium (RPE) is closely connected with the underlying choroid and the upper retinal nerve cells. Its main functions may include: controlling the fluids and nutritions in the subretinal space; functioning as a blood-retinal barrier; synthesis of the growth factor for adjusting the local structure; absorption of lights and regulation of the electrical balance; regeneration and synthesis of visual pigments; phagocytosis and digestion of photoreceptor outer segments; maintenance of retinal attachment; and regeneration and repair after injury. RPE is generally considered to be an important tissue for maintaining the photoreceptor function, and is also affected by many lesions in the choroid and retina.

As used herein, the term "retinal pigment epithelium (RPE) atrophy" generally refers to degenerative changes in the retinal pigment epithelium (RPE) manifested by cell death or dysfunction. The age-related macular degeneration or retinitis pigmentosa (RP) is often accompanied by the retinal pigment epithelium atrophy. The retinitis pigmentosa (abbreviated as RP), also known as the retinal pigment lesion, usually refers to a class of inherited ocular diseases. There are three modes of inheritance: autosomal recessive, dominant, and X-linked, and dihybrid inheritance and mitochondrial inheritance are also present. The common symptoms in the early stage may be night blindness, narrowing of visual field, the ability to see the scene right ahead but the inability to see the visual field slightly to the left and right, and then the gradual disappearance of vision. RP may include uniocular primary retinitis pigmentosa, sector primary retinitis pigmentosa, central or paracentral primary retinitis pigmentosa, retinitis pigmentosa sine pigmento, albescent punctate degeneration of retina, Bietti's crystalline dystrophy, pigmented paravenous retinitis pigmentosa, preserved para-arteriolar retinal pigment epithelium retinitis pigmentosa, Leber congenital amaurosis, and retinitis pigmentosa in other syndromes.

As used herein, the term "Bietti's crystalline dystrophy (BCD)" generally refers to a class of autosomal recessive ocular diseases first described in 1937 by an Italian ophthalmologist, Dr. GB Bietti. The symptoms mainly include crystals (transparent coverings) in the cornea; small, yellow or white, crystalline deposits deposited in the photosensitive tissues of the retina; and progressive atrophy of the retina, choriocapillary, and choroid. The Bietti's crystalline dystrophy may include a disease caused by CYP4V2 gene mutation.

As used herein, the term "comprise" or "include" generally means the inclusion of expressly specified features, but without the exclusion of other elements.

As used herein, the term "about" generally refers to variations above or below the specified value within the range of 0.5%-10%, such as variations above or below the specified value within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

In one aspect, the present application provides the use of CYP4V2 and RdCVF in the manufacture of a medicament for treating, alleviating, and/or preventing a disease or disorder associated with retinal pigment epithelium (RPE) atrophy.

In the present application, the medicament may comprise a polynucleotide encoding CYP4V2 and a polynucleotide encoding RdCVF. For example, the polynucleotide encoding CYP4V2 and the polynucleotide encoding RdCVF are in different vectors. For example, the polynucleotide encoding CYP4V2 and the polynucleotide encoding RdCVF are in the same vector.

In another aspect, the present application also provides a vector combination for treating, alleviating, and/or preventing a disease or disorder associated with retinal pigment epithelium (RPE) atrophy, comprising a first vector and a second vector, wherein the first vector may comprise a polynucleotide encoding CYP4V2, and the second vector may comprise a polynucleotide encoding RdCVF.

In another aspect, the present application also provides an isolated nucleic acid molecule, wherein the isolated nucleic acid molecule may comprise a polynucleotide encoding CYP4V2 and a polynucleotide encoding RdCVF.

In another aspect, the present application also provides a cell, wherein the cell may comprise the nucleic acid molecule described herein or the vector combination described herein.

The present application also provides a pharmaceutical composition, wherein the pharmaceutical composition may comprise the nucleic acid molecule described herein, the vector combination described herein, and/or the cell described herein.

In another aspect, the present application also provides the use of the nucleic acid molecule described herein, the vector combination described herein, the cell described herein, and/or the pharmaceutical composition described herein in the manufacture of a medicament, wherein the medicament may be used in for preventing, alleviating, and/or treating a disease or disorder associated with retinal pigment epithelium (RPE) atrophy.

### CYP4V2

In the present application, CYP4V2 may comprise a class of proteins whose dysfunctions or encoding gene mutations may lead to Bietti's crystalline dystrophy, including but not limited to CYP4V2 in human, chimpanzee, gorilla, rhesus monkey, dog, cow, mouse, rat, chicken, drosophila, nematode, or frog, or functional variants thereof.

For example, the CYP4V2 may include human CYP4V2.

For example, the CYP4V2 may comprise an amino acid sequence set forth in any of SEQ ID NOs: 76-82.

For example, the CYP4V2 may comprise an amino acid sequence set forth in SEQ ID NO: 76.

For example, the CYP4V2 may comprise an amino acid sequence having at least 90% identity, e.g., any amino acid sequence having at least 95%, at least 96%, at least 97%, at least 98%, or at least 99% identity, to the amino acid sequence set forth in SEQ ID NOs: 76-82, and the combined administration of said amino acid sequence and RdCVF described herein can ameliorate the disease or disorder associated with retinal pigment epithelium (RPE) atrophy.

In the present application, the polynucleotide encoding CYP4V2 may comprise a nucleic acid sequence set forth in any of SEQ ID NOs: 62-68.

For example, the polynucleotide encoding CYP4V2 may comprise a nucleic acid sequence set forth in SEQ ID NO: 62.

For example, the polynucleotide encoding CYP4V2 may comprise an amino acid sequence having at least 90% identity, e.g., any polynucleotide sequence having at least 95%, at least 96%, at least 97%, at least 98%, or at least 99% identity, to the nucleic acid sequence set forth in SEQ ID NOs: 62-82, and the combined administration of a polypeptide encoded by said nucleotide sequence and RdCVF described herein can ameliorate the disease or disorder associated with retinal pigment epithelium (RPE) atrophy.

### RdCVF

In the present application, the RdCVF may include a protein produced by rod cells and beneficial for cone cells. The RdCVF includes, but not limited to, RdCVF in human, chimpanzee, gorilla, rhesus monkey, dog, cow, rat, mouse, chicken, zebrafish, and frog, or functional variants thereof.

For example, the RdCVF may include human RdCVF.

For example, the RdCVF may comprise an amino acid sequence set forth in any of SEQ ID NOs: 83-89.

For example, the RdCVF may comprise an amino acid sequence set forth in SEQ ID NO: 83.

For example, the RdCVF may comprise an amino acid sequence having at least 90% identity, e.g., any amino acid sequence having at least 95%, at least 96%, at least 97%, at least 98%, or at least 99% identity, to the amino acid sequence set forth in SEQ ID NOs: 83-89, and the combined administration of said amino acid sequence and CYP4V2 described herein can ameliorate the disease or disorder associated with retinal pigment epithelium (RPE) atrophy.

In the present application, the polynucleotide encoding RdCVF may comprise a nucleic acid sequence set forth in any of SEQ ID NOs: 69-75.

For example, the polynucleotide encoding RdCVF may comprise a nucleic acid sequence set forth in SEQ ID NO: 69.

For example, the polynucleotide encoding RdCVF may comprise an amino acid sequence having at least 90% identity, e.g., any polynucleotide sequence having at least 95%, at least 96%, at least 97%, at least 98%, or at least 99% identity, to the nucleic acid sequence set forth in SEQ ID NOs: 69-75, and the combined administration of a polypeptide encoded by said nucleotide sequence and CYP4V2 described herein can ameliorate the disease or disorder associated with retinal pigment epithelium (RPE) atrophy.

### Promoter

In the present application, the promoter may include a RPE cell-specific promoter, retinal cell-specific promoter, corneal cell-specific promoter, ocular cell-specific promoter, or constitutive promoter. The promoter may also include a mammalian beta-actin promoter or a viral promoter. The promoter may also include a CAG promoter (hybrid CMV early enhancer/chicken beta actin promoter, also known as CAGGS promoter, CB promoter, or CBA promoter), human beta actin promoter, small CBA (smCBA) promoter, CBS promoter or CBh promoter, elongation factor 1α short (EFS) promoter, elongation factor 1α (EF-1α) promoter, CMV promoter, PGK promoter, UBC promoter, GUSB promoter, UCOE promoter, VMD2 (also known as BEST1) promoter, OPEFS promoter, CYP4V2 native promoter, RPE65 promoter, or hybrids or derivatives thereof.

For example, the promoter may include a CYP4V2 native promoter, and the CYP4V2 native promoter may comprise all or part of 2000 bp nucleotide sequence upstream of the polynucleotide encoding CYP4V2.

For example, the promoter may comprise a nucleotide sequence set forth in any of SEQ ID NOs: 1-12.

For example, the CYP4V2 native promoter may include CYP4V2-Pf promoter, CYP4V2-P1 promoter, CYP4V2-P2 promoter, CYP4V2-P3 promoter, CYP4V2-P4 promoter, CYP4V2-P5 promoter, and CYP4V2-P6 promoter; the CYP4V2-Pf promoter may comprise a nucleic acid sequence set forth in SEQ ID NO: 6, the CYP4V2-P1 promoter may comprise a nucleic acid sequence set forth in SEQ ID NO: 7, the CYP4V2-P2 promoter may comprise a nucleic acid sequence set forth in SEQ ID NO: 8, the CYP4V2-P3 promoter may comprise a nucleic acid sequence set forth in SEQ ID NO: 9, the CYP4V2-P4 promoter may comprise a nucleic acid sequence set forth in SEQ ID NO: 10, the CYP4V2-P5 promoter may comprise a nucleic acid sequence set forth in SEQ ID NO: 11, the CYP4V2-P6 promoter may comprise a nucleic acid sequence set forth in SEQ ID NO: 12.

For example, the promoter may be OPEFS, comprising a nucleic acid sequence set forth in SEQ ID NO: 1.

For example, the promoter may be EFS, comprising a nucleic acid sequence set forth in SEQ ID NO: 2.

For example, the promoter may be EF1a, comprising a nucleic acid sequence set forth in SEQ ID NO: 3.

For example, the promoter may be CAG, comprising a nucleic acid sequence set forth in SEQ ID NO: 4.

For example, the promoter may be RPE65, comprising a nucleic acid sequence set forth in SEQ ID NO: 5.

### PolyA signal site

In the present application, the PolyA signal site may include SV40 signal site, BGH signal site, WPRE signal site, WPRE-SV40 signal site, WPRE-BGH signal site, or derivatives thereof.

For example, the PolyA signal site can be recognized by a polyadenylation-related cleavage factor, leading to SV40 PolyA sequence, BGH signal PolyA sequence, HSV signal PolyA sequence, TK signal PolyA sequence, WPRE signal PolyA sequence, etc.

For example, the PolyA signal site may comprise AAUAAA sequence.

For example, the PolyA signal site may comprise a nucleic acid sequence set forth in SEQ ID NOs: 17-21.

### Self-cleaving peptide

In the present application, the polynucleotide encoding the self-cleaving peptide is located between the polynucleotide encoding CYP4V2 and the polynucleotide encoding RdCVF.

For example, the self-cleaving peptide may include T2A, P2A, E2A, or F2A.

For example, the self-cleaving peptide may include P2A.

For example, the self-cleaving peptide may comprise an amino acid sequence set forth in any of SEQ ID NOs: 26-29.

For example, the polynucleotide encoding the self-cleaving peptide may comprise a nucleic acid sequence set forth in any of SEQ ID NOs: 22-25.

For example, a GSG (Gly-Ser-Gly, glycine, serine, glycine) sequence can also be linked to the N-terminus of the cleaving peptide.

### Intron

In the present application, an intron may be located at 5' end of the target gene, or located in the nucleotide sequence of the target gene. The target gene may include a polynucleotide encoding CYP4V2 or a polynucleotide encoding RdCVF. The intron can enhance the expression of the target gene. For example, the intron may include human β-globin intron, SV40 intron, hybrid CBA/MVM intron, or other artificially synthesized introns.

For example, the intron comprises a nucleotide sequence set forth in any of SEQ ID NOs: 13-16.

### Isolated nucleic acid molecule

In the present application, the isolated nucleic acid molecule may sequentially comprise, from 5' end to 3' end, a polynucleotide encoding CYP4V2, and a polynucleotide encoding RdCVF.

For example, the isolated nucleic acid molecule may sequentially comprise, from 5' end to 3' end, a polynucleotide encoding CYP4V2, and a polynucleotide encoding RdCVF, wherein the polynucleotide encoding CYP4V2 may comprise a nucleic acid sequence set forth in any of SEQ ID NOs: 62-68, and the polynucleotide encoding RdCVF may comprise a nucleic acid sequence set forth in any of SEQ ID NOs: 69-75.

In the present application, the isolated nucleic acid molecule may sequentially comprise, from 5' end to 3' end, a polynucleotide encoding RdCVF, and a polynucleotide encoding CYP4V2.

For example, the isolated nucleic acid molecule may sequentially comprise, from 5' end to 3' end, a polynucleotide encoding RdCVF, and a polynucleotide encoding CYP4V2, wherein the polynucleotide encoding RdCVF may comprise a nucleic acid sequence set forth in any of SEQ ID NOs: 69-75, and the polynucleotide encoding CYP4V2 may comprise a nucleic acid sequence set forth in any of SEQ ID NOs: 62-68.

In the present application, the isolated nucleic acid molecule may also comprise a promoter.

For example, the promoter is located at 5' end of the polynucleotide encoding CYP4V2 and operably linked to the polynucleotide encoding CYP4V2.

For example, the promoter is located at 5' end of the polynucleotide encoding RdCVF and operably linked to the polynucleotide encoding RdCVF.

For example, the isolated nucleic acid molecule may sequentially comprise, from 5' end to 3' end, a promoter, a polynucleotide encoding CYP4V2, and a polynucleotide encoding RdCVF.

For example, the isolated nucleic acid molecule may sequentially comprise, from 5' end to 3' end, a promoter, a polynucleotide encoding RdCVF, and a polynucleotide encoding CYP4V2.

For example, the promoter may comprise a nucleic acid sequence set forth in any of SEQ ID NOs: 1-12, the polynucleotide encoding RdCVF may comprise a nucleic acid sequence set forth in any of SEQ ID NOs: 69-75, and the polynucleotide encoding CYP4V2 may comprise a nucleic acid sequence set forth in any of SEQ ID NOs: 62-68.

For example, the isolated nucleic acid molecule may sequentially comprise, from 5' end to 3' end, a promoter, a polynucleotide encoding CYP4V2, a promoter, and a polynucleotide encoding RdCVF.

For example, the isolated nucleic acid molecule may sequentially comprise, from 5' end to 3' end, a promoter, a polynucleotide encoding CYP4V2, a promoter, and a polynucleotide encoding RdCVF.

For example, the promoter may comprise a nucleic acid sequence set forth in any of SEQ ID NOs: 1-12, the polynucleotide encoding CYP4V2 may comprise a nucleic acid sequence set forth in any of SEQ ID NOs: 62-68, and the polynucleotide encoding RdCVF may comprise a nucleic acid sequence set forth in any of SEQ ID NOs: 69-75.

For example, the isolated nucleic acid molecule may sequentially comprise, from 5' end to 3' end, a promoter, a polynucleotide encoding RdCVF, a promoter, and a polynucleotide encoding CYP4V2.

For example, the promoter may comprise a nucleic acid sequence set forth in any of SEQ ID NOs: 1-12, the polynucleotide encoding RdCVF may comprise a nucleic acid sequence set forth in any of SEQ ID NOs: 69-75, and the polynucleotide encoding CYP4V2 may comprise a nucleic acid sequence set forth in any of SEQ ID NOs: 62-68.

In the present application, the isolated nucleic acid molecule may also comprise a polynucleotide encoding a self-cleaving peptide.

For example, the isolated nucleic acid molecule may sequentially comprise, from 5' end to 3' end, a promoter, a polynucleotide encoding CYP4V2, a polynucleotide encoding a self-cleaving peptide, and a polynucleotide encoding RdCVF.

For example, the promoter may comprise a nucleic acid sequence set forth in any of SEQ ID NOs: 1-12, the polynucleotide encoding CYP4V2 may comprise a nucleic acid sequence set forth in any of SEQ ID NOs: 62-68, the polynucleotide encoding the self-cleaving peptide may comprise a nucleic acid sequence set forth in any of SEQ ID NOs: 22-25, and the polynucleotide encoding RdCVF may comprise a nucleic acid sequence set forth in any of SEQ ID NOs: 69-75.

For example, the isolated nucleic acid molecule may sequentially comprise, from 5' end to 3' end, a promoter, a polynucleotide encoding RdCVF, a polynucleotide encoding a self-cleaving peptide, and a polynucleotide encoding CYP4V2.

For example, the promoter may comprise a nucleic acid sequence set forth in any of SEQ ID NOs: 1-12, the polynucleotide encoding RdCVF may comprise a nucleic acid sequence set forth in any of SEQ ID NOs: 69-75, the polynucleotide encoding the self-cleaving peptide may comprise a nucleic acid sequence set forth in any of SEQ ID NOs: 22-25, and the polynucleotide encoding CYP4V2 may comprise a nucleic acid sequence set forth in any of SEQ ID NOs: 62-68.

For example, the isolated nucleic acid molecule may comprise a nucleic acid sequence set forth in any of SEQ ID NOs: 101-105.

In the present application, the isolated nucleic acid molecule may also comprise a PolyA signal site located at 3' end of the polynucleotide encoding CYP4V2.

In the present application, the isolated nucleic acid molecule may also comprise a PolyA signal site located at 3' end of the polynucleotide encoding RdCVF.

For example, the isolated nucleic acid molecule may sequentially comprise, in 5' to 3' direction, a promoter, a polynucleotide encoding CYP4V2, a polynucleotide encoding a self-cleaving peptide, a polynucleotide encoding RdCVF, and a PolyA signal site.

For example, the promoter may comprise a nucleic acid sequence set forth in any of SEQ ID NOs: 1-12, the polynucleotide encoding RdCVF may comprise a nucleic acid sequence set forth in any of SEQ ID NOs: 69-75, the polynucleotide encoding the self-cleaving peptide may comprise a nucleic acid sequence set forth in any of SEQ ID NOs: 22-25, the polynucleotide encoding CYP4V2 may comprise a nucleic acid sequence set forth in any of SEQ ID NOs: 62-68, and the PolyA signal site may comprise a nucleotide sequence set forth in any of SEQ ID NOs: 17-21.

In the present application, the isolated nucleic acid molecule may sequentially comprise, in 5' to 3' direction, a promoter, a polynucleotide encoding RdCVF, a polynucleotide encoding a self-cleaving peptide, a polynucleotide encoding CYP4V2, and a PolyA signal site.

For example, the promoter may comprise a nucleic acid sequence set forth in any of SEQ ID NOs: 1-12, the polynucleotide encoding RdCVF may comprise a nucleic acid sequence set forth in any of SEQ ID NOs: 69-75, the polynucleotide encoding the self-cleaving peptide may comprise a nucleic acid sequence set forth in any of SEQ ID NOs: 22-25, the polynucleotide encoding CYP4V2 may comprise a nucleic acid sequence set forth in any of SEQ ID NOs: 62-68, and the PolyA signal site may comprise a nucleotide sequence set forth in any of SEQ ID NOs: 17-21.

For example, the isolated nucleic acid molecule may comprise a nucleic acid sequence set forth in any of SEQ ID NOs: 106-108.

In the present application, the isolated nucleic acid molecule may also comprise an intron.

For example, the intron may comprise a nucleotide sequence set forth in any of SEQ ID NOs: 13-16.

For example, the intron is located in the polynucleotide encoding CYP4V2, or located at 5' end of the polynucleotide encoding CYP4V2.

For example, the isolated nucleic acid molecule may sequentially comprise, in 5' to 3' direction, a promoter, an intron, a polynucleotide encoding CYP4V2, a polynucleotide encoding a self-cleaving peptide, and a polynucleotide encoding RdCVF.

For example, the isolated nucleic acid molecule may sequentially comprise, in 5' to 3' direction, a promoter, a polynucleotide encoding CYP4V2 with the intron inserted, a polynucleotide encoding a self-cleaving peptide, and a polynucleotide encoding RdCVF.

For example, the promoter may comprise a nucleic acid sequence set forth in any of SEQ ID NOs: 1-12, the intron may comprise a nucleic acid sequence set forth in any of SEQ ID NOs: 13-16, the polynucleotide encoding CYP4V2 may comprise a nucleic acid sequence set forth in any of SEQ ID NOs: 62-68, the polynucleotide encoding the self-cleaving peptide may comprise a nucleic acid sequence set forth in any of SEQ ID NOs: 22-25, and the polynucleotide encoding RdCVF may comprise a nucleic acid sequence set forth in any of SEQ ID NOs: 69-75.

For example, the isolated nucleic acid molecule may sequentially comprise, in 5' to 3' direction, a promoter, an intron, a polynucleotide encoding CYP4V2, a polynucleotide encoding a self-cleaving peptide, a polynucleotide encoding RdCVF, and a PolyA signal site.

For example, the isolated nucleic acid molecule may sequentially comprise, in 5' to 3' direction, a promoter, a polynucleotide encoding CYP4V2 with the intron inserted, a polynucleotide encoding a self-cleaving peptide, a polynucleotide encoding RdCVF, and a PolyA signal site.

For example, the promoter may comprise a nucleic acid sequence set forth in any of SEQ ID NOs: 1-12, the intron may comprise a nucleic acid sequence set forth in any of SEQ ID NOs: 13-16, the polynucleotide encoding CYP4V2 may comprise a nucleic acid sequence set forth in any of SEQ ID NOs: 62-68, the polynucleotide encoding the self-cleaving peptide may comprise a nucleic acid sequence set forth in any of SEQ ID NOs: 22-25, the polynucleotide encoding RdCVF may comprise a nucleic acid sequence set forth in any of SEQ ID NOs: 69-75, and the PolyA signal site may comprise a nucleotide sequence set forth in any of SEQ ID NOs: 17-21.

For example, the polynucleotide encoding CYP4V2 with the intron inserted may comprise a nucleic acid sequence set forth in SEQ ID NO: 116.

For example, the intron is located in the polynucleotide encoding RdCVF, or located at 5' end of the polynucleotide encoding RdCVF.

For example, the isolated nucleic acid molecule may sequentially comprise, in 5' to 3' direction, a promoter, an intron, a polynucleotide encoding RdCVF, a polynucleotide encoding a self-cleaving peptide, and a polynucleotide encoding RdCVF.

For example, the isolated nucleic acid molecule may sequentially comprise, in 5' to 3' direction, a promoter, a polynucleotide encoding RdCVF with the intron inserted, a polynucleotide encoding a self-cleaving peptide, and a polynucleotide encoding CYP4V2.

For example, the promoter may comprise a nucleic acid sequence set forth in any of SEQ ID NOs: 1-12, the intron may comprise a nucleic acid sequence set forth in any of SEQ ID NOs: 13-16, the polynucleotide encoding RdCVF may comprise a nucleic acid sequence set forth in any of SEQ ID NOs: 69-75, the polynucleotide encoding the self-cleaving peptide may comprise a nucleic acid sequence set forth in any of SEQ ID NOs: 22-25, and the polynucleotide encoding CYP4V2 may comprise a nucleic acid sequence set forth in any of SEQ ID NOs: 62-68.

For example, the isolated nucleic acid molecule may sequentially comprise, in 5' to 3' direction, a promoter, an intron, a polynucleotide encoding RdCVF, a polynucleotide encoding a self-cleaving peptide, a polynucleotide encoding CYP4V2, and a PolyA signal site.

For example, the isolated nucleic acid molecule may sequentially comprise, in 5' to 3' direction, a promoter, a polynucleotide encoding RdCVF with the intron inserted, a polynucleotide encoding a self-cleaving peptide, a polynucleotide encoding CYP4V2, and a PolyA signal site.

For example, the promoter may comprise a nucleic acid sequence set forth in any of SEQ ID NOs: 1-12, the intron may comprise a nucleic acid sequence set forth in any of SEQ ID NOs: 13-16, the polynucleotide encoding RdCVF may comprise a nucleic acid sequence set forth in any of SEQ ID NOs: 69-75, the polynucleotide encoding the self-cleaving peptide may comprise a nucleic acid sequence set forth in any of SEQ ID NOs: 22-25, the polynucleotide encoding CYP4V2 may comprise a nucleic acid sequence set forth in any of SEQ ID NOs: 62-68, and the PolyA signal site may comprise a nucleotide sequence set forth in any of SEQ ID NOs: 17-21.

For example, the isolated nucleic acid molecule may comprise a nucleotide sequence set forth in any of SEQ ID NOs: 109-115.

### Vector

In the present application, the vector may include plasmid, phagemid, cosmid, artificial chromosome (such as yeast artificial chromosome (YAC), bacterial artificial chromosome (BAC), or P1-derived artificial chromosome (PAC)), phage (such as λ phage or M13 phage), and viral vectors.

For example, the viral vector may include retrovirus (including lentivirus), adenovirus, adeno-associated virus (AAV vector), herpesvirus (such as herpes simplex virus), poxvirus, baculovirus, papillomavirus, and papovavirus (such as SV40) vectors.

For example, the adeno-associated virus vector (AAV vector) gene may comprise an inverted terminal repeat (ITR) and an open reading frame (ORF), wherein the open reading frame may include a polynucleotide encoding Rep protein, and may also include a polynucleotide encoding a capsid.

For example, the adeno-associated virus vector (AAV vector) may also include a recombinant adeno-associated virus vector (rAAV vector).

For example, the capsid, ITR, and other selected AAV components in the recombinant adeno-associated virus vector can be selected from any AAV, including but not limited to, AAV1, AAV2, AAV3, AAV4, AAV5, AAV6, AAV7, AAV8, AAV9, AAV8bp, AAV7M8 and AAVAnc80, DJ, DJ/8, Rh10, variants of any known or mentioned AAV, or variants or mixtures thereof.

For example, the AAV vector may be any one of AAV1, AAV2, AAV3, AAV4, AAV5, AAV6, AAV7, AAV8, AAV9, AAV8bp, AAV7M8, AAVAnc80, DJ, DJ/8, and Rh10.

For example, the AAV vector is an AAV vector having eye tissue-affinity, e.g., AAV2, AAV3, AAV4, AAV5, AAV8, DJ/8, or any rAAV vector.

For example, the AAV vector may be AAV2/2, AAV2/5, AAV2/8, or AAV2/9.

For example, the viral vector may include pAAV-RC5-Amp, RC8-cap, AAV2/8, AAV-helper-Amp, and AAV-helper.

For example, the viral vector may comprise a nucleotide sequence set forth in any of SEQ ID NOs: 133-137.

For example, the vector may also comprise a nucleotide sequence set forth in any of SEQ ID NOs: 90-115.

For example, the vector may also comprise a nucleotide sequence set forth in any of SEQ ID NOs: 117-121.

For example, the vector may comprise a nucleotide sequence set forth in any of SEQ ID NOs: 122-132.

In the present application, the vector may comprise the isolated nucleic acid molecule described herein.

For example, the vector may comprise a nucleotide sequence set forth in any of SEQ ID NOs: 101-115.

In the present application, the vector may comprise a polynucleotide encoding CYP4V2 or a polynucleotide encoding RdCVF.

For example, the vector may also comprise a promoter located at 5' end of the polynucleotide encoding CYP4V2 and operably linked to the polynucleotide encoding CYP4V2.

For example, the vector may also comprise a promoter located at 5' end of the polynucleotide encoding RdCVF and operably linked to the polynucleotide encoding RdCVF.

For example, the vector may also comprise a PolyA signal site located at 3' end of the polynucleotide encoding CYP4V2.

For example, the vector may sequentially comprise, in 5' to 3' direction: a promoter, a polynucleotide encoding CYP4V2, and a PolyA signal site.

For example, the vector may sequentially comprise, in 5' to 3' direction: a promoter sequence set forth in any of SEQ ID NOs: 1-12, a polynucleotide sequence encoding CYP4V2 set forth in any of SEQ ID NOs: 62-68, a PolyA signal site sequence set forth in any of SEQ ID NOs: 17-21.

For example, the vector may comprise a nucleotide sequence set forth in any of SEQ ID NOs: 90-92.

For example, the vector may also comprise a PolyA signal site located at 3' end of the polynucleotide encoding RdCVF.

For example, the vector may sequentially comprise, in 5' to 3' direction: a promoter, a polynucleotide encoding RdCVF, and a PolyA signal site.

For example, the vector may sequentially comprise, in 5' to 3' direction: a promoter sequence set forth in any of SEQ ID NOs: 1-12, a polynucleotide sequence encoding RdCVF set forth in any of SEQ ID NOs: 69-75, a PolyA signal site sequence set forth in any of SEQ ID NOs: 17-21.

For example, the vector may comprise a nucleic acid sequence set forth in any of SEQ ID NOs: 96-98.

For example, the vector may also comprise an intron.

For example, the intron is located in the polynucleotide encoding CYP4V2, or located at 5' end of the polynucleotide encoding CYP4V2.

For example, the intron is located in the polynucleotide encoding RdCVF, or located at 5' end of the polynucleotide encoding RdCVF.

For example, the vector may sequentially comprise, in 5' to 3' direction: a promoter, an intron, and a polynucleotide encoding CYP4V2 or a polynucleotide encoding RdCVF.

For example, the vector may sequentially comprise, in 5' to 3' direction: a promoter, and a polynucleotide encoding CYP4V2 with the intron inserted or a polynucleotide encoding RdCVF with the intron inserted.

For example, the vector may sequentially comprise, in 5' to 3' direction: a promoter, an intron, a polynucleotide encoding CYP4V2 or a polynucleotide encoding RdCVF, and a PolyA signal site.

For example, the vector may sequentially comprise, in 5' to 3' direction: a promoter, a polynucleotide encoding CYP4V2 with the intron inserted or a polynucleotide encoding RdCVF with the intron inserted, and a PolyA signal site.

For example, the promoter may comprise a nucleic acid sequence set forth in any of SEQ ID NOs: 1-12, the intron may comprise a nucleic acid sequence set forth in any of SEQ ID NOs: 13-16, the polynucleotide encoding CYP4V2 may comprise a nucleic acid sequence set forth in any of SEQ ID NOs: 62-68, the polynucleotide encoding RdCVF may comprise a nucleic acid sequence set forth in any of SEQ ID NOs: 69-75, and the PolyA signal site may comprise a nucleotide sequence set forth in any of SEQ ID NOs: 17-21.

For example, the polynucleotide encoding CYP4V2 with the intron inserted may comprise a nucleic acid sequence set forth in SEQ ID NO: 116.

For example, the vector may comprise a nucleotide sequence set forth in any of SEQ ID NOs: 93-95 and 99-100.

### Vector combination

The present application also provides a vector combination for treating, alleviating, and/or preventing a disease or disorder associated with retinal pigment epithelium (RPE) atrophy, comprising a first vector and a second vector, wherein the first vector may comprise a polynucleotide encoding CYP4V2, and the second vector may comprise a polynucleotide encoding RdCVF.

For example, the first vector may also comprise a promoter located at 5' end of the polynucleotide encoding CYP4V2 and operably linked to the polynucleotide encoding CYP4V2.

For example, the first vector may sequentially comprise, in 5' to 3' direction: a promoter sequence set forth in any of SEQ ID NOs: 1-12, and a polynucleotide sequence encoding CYP4V2 set forth in any of SEQ ID NOs: 62-68.

For example, the first vector may comprise a polynucleotide sequence set forth in SEQ ID NO: 117.

For example, the second vector may also comprise a promoter located at 5' end of the polynucleotide encoding RdCVF and operably linked to the polynucleotide encoding RdCVF.

For example, the second vector may comprise a polynucleotide sequence set forth in SEQ ID NO: 120.

For example, the first vector may sequentially comprise, in 5' to 3' direction: a promoter sequence set forth in any of SEQ ID NOs: 1-12, and a polynucleotide sequence encoding RdCVF set forth in any of SEQ ID NOs: 69-75.

For example, the first vector may also comprise a PolyA signal site located at 3' end of the polynucleotide encoding CYP4V2.

For example, the second vector may also comprise a PolyA signal site located at 3' end of the polynucleotide encoding RdCVF.

For example, the first vector may sequentially comprise, in 5' to 3' direction: a promoter, a polynucleotide encoding CYP4V2, and a PolyA signal site; and/or the second vector may sequentially comprise, in 5' to 3' direction: a promoter, a polynucleotide encoding RdCVF, and a PolyA signal site.

For example, the first vector may sequentially comprise, in 5' to 3' direction: a promoter sequence set forth in any of SEQ ID NOs: 1-12, a polynucleotide sequence encoding CYP4V2 set forth in any of SEQ ID NOs: 62-68, and a PolyA signal site sequence set forth in any of SEQ ID NOs: 17-21.

For example, the first vector may comprise a nucleotide sequence set forth in any of SEQ ID NOs: 90-92.

For example, the second vector may sequentially comprise, in 5' to 3' direction: a promoter sequence set forth in any of SEQ ID NOs: 1-12, a polynucleotide sequence encoding RdCVF set forth in any of SEQ ID NOs: 69-75, a PolyA signal site sequence set forth in any of SEQ ID NOs: 17-21.

For example, the second vector may comprise a nucleotide sequence set forth in any of SEQ ID NOs: 96-98.

For example, the first vector and/or the second vector may also comprise an intron(s).

For example, the intron is located in the polynucleotide encoding CYP4V2, or located at 5' end of the polynucleotide encoding CYP4V2.

For example, the intron is located in the polynucleotide encoding RdCVF, or located at 5' end of the polynucleotide encoding RdCVF.

For example, the first vector may comprise a nucleotide sequence set forth in any of SEQ ID NOs: 93-95; and/or the second vector may comprise a nucleotide sequence set forth in any of SEQ ID NOs: 99-100.

For example, the first vector may comprise a nucleotide sequence set forth in any of SEQ ID NOs: 118-119; and/or the second vector may comprise a nucleotide sequence set forth in any of SEQ ID NO: 121.

### Cell

The present application also provides a cell, wherein the cell may comprise the nucleic acid molecule described herein or the vector combination described herein.

For example, the cell may be a cell in which the nucleic acid molecule is expressed.

For example, the cell may include the progeny of a single cell. The progeny may not necessarily be completely identical to the original parent cell (either morphologically in total DNA complement, or genomically).

For example, the cell may also include a cell transfected in vitro with the vector described herein.

For example, the cell may include bacterial cell (e.g., *E. coli*), yeast cell, or other eukaryotic cells, such as COS cell, Chinese hamster ovary (CHO) cell, HeLa cell, HEK293 cell, COS-1 cell, NS0 cell or myeloma cell, and 293T cell.

For example, the cell is a cell from a patient with Bietti's crystalline dystrophy (BCD).

For example, the cell may include somatic or stem cell.

For example, the cell may include retinal cell, corneal cell, choroidal cell, lens cell, nerve cell, RPE cell, and stem cell, and the stem cell may include induced pluripotent stem cell (iPSC), embryonic stem cell (ESC), mesenchymal stem cell (MSC), adult stem cell, or any cell differentiated from stem cell.

For example, the retinal cell, corneal cell, choroidal cell, lens cell, nerve cell, or RPE cell can be induced and differentiated from the stem cell.

For example, the cell may include ARPE-19 cell, or human iPSC-induced RPE cell.

### Pharmaceutical composition

The present application also provides a pharmaceutical composition, wherein the pharmaceutical composition may comprise the nucleic acid molecule described herein, the vector combination described herein, and/or the cell described herein.

For example, the pharmaceutical composition may also optionally comprise a pharmaceutically acceptable adjuvant.

For example, the pharmaceutical composition may also comprise one or more (pharmaceutically effective) vehicles, stabilizers, excipients, diluents, solubilizers, surfactants, emulsifiers, and/or preservatives for suitable formulations.

For example, the acceptable ingredients of the composition are not toxic to recipients at the dosages and concentrations employed.

For example, the pharmaceutical composition includes, but is not limited to, liquid, frozen, and lyophilized compositions.

For example, the pharmaceutically acceptable adjuvant may include any and all solvents, dispersion media, coatings, isotonic agents, and absorption delaying agents compatible with the pharmaceutical administration, which are generally safe, non-toxic, and neither biologically nor otherwise undesirable.

For example, the pharmaceutical composition may involve parenteral, transdermal, intracavity, intraarterial, intrathecal, and/or intraocular administration, or direct injection into tissues.

For example, the pharmaceutical composition may be administrated to a patient or subject by instillation, infusion, or injection.

For example, the pharmaceutical composition may be uninterruptedly (or continuously) administrated.

For example, the uninterrupted (or continuous) administration may be achieved by a small pump system worn by a patient for measuring the influx of the therapeutic agent into the patient, as described in WO2015/036583.

In the present application, the subject may include human and non-human animals. For example, the subject may include, but not limited to, cats, dogs, horses, pigs, cows, sheep, rabbits, mice, rats, or monkeys.

### Disease or disorder associated with retinal pigment epithelium (RPE) atrophy

In the present application, the disease or disorder associated with retinal pigment epithelium (RPE) atrophy may include age-related macular degeneration or retinitis pigmentosa (RP).

For example, the retinitis pigmentosa may include uniocular primary retinitis pigmentosa, sector primary retinitis pigmentosa, central or paracentral primary retinitis pigmentosa, retinitis pigmentosa sine pigmento, albescent punctate degeneration of retina, Bietti's crystalline dystrophy (BCD), pigmented paravenous retinitis pigmentosa, preserved para-arteriolar retinal pigment epithelium retinitis pigmentosa, Leber congenital amaurosis, and retinitis pigmentosa in other syndromes.

For example, the retinitis pigmentosa may include Bietti's crystalline dystrophy.

For example, the Bietti's crystalline dystrophy may include a disease caused by CYP4V2 gene mutation.

For example, the CYP4V2 gene mutation may include, but not limited to, missense mutation, replication error, splice site error, frameshift, base deletion or insertion, nonsense mutation, polymorphism (e.g., single nucleotide polymorphism), premature termination, partial or whole deletion of CYP4V2 gene, and unidentified CYP4V2 gene variations associated with Bietti's crystalline dystrophy.

For example, the CYP4V2 gene mutation may include the mutations shown in Table 1:

**Table 1 Certain types of CYP4V2 gene mutations**

| **Exon or intron position** | **Nucleic acid change** | **Predicted protein change** |
|---|---|---|
| **1** | **c.31C>T** | **p.Q11X** |
| **1** | **c.64C>G** | **p.L22V** |
| **1** | **c.71T >C** | **p.L24P** |
| **1** | **c.77G >A** | **p.G26D** |
| **1** | **c.l30T>A** | **p.W44R** |
| **1** | **c.l34A>C** | **p.Q45P** |
| **1** | **c.l81G>A** | **p.G61S** |
| **1** | **c. l97T>G** | **p.M66R** |
| **IVS1** | **c.214+lG>A** | **Exon 1 deletion** |
| **IVS1** | **c.214+25delT** | **Unknown** |
| **IVS1** | **c.215-2A>G** | **Exon 2 deletion** |
| **IVS1** | **c.215-lG>A** | **Exon 2 deletion** |
| **2** | **c.219T>A** | **p.F73L** |
| **2** | **c.237G>T** | **p.E79D** |
| **2** | **c.253C>T** | **p.R85C** |
| **2** | **c.277T>C** | **p.W93R** |
| **2** | **c.283G>A** | **p.G95R** |
| **2** | **c.327G>A** | **Unknown** |
| **IVS2** | **c.327+lG>A** | **p.E72Gfs*5** |
| **IVS2** | **c.327+llG>C** | **Unknown** |
| **3** | **c.332T>C** | **p.I111T** |
| **3** | **c.335T>G** | **p.L112*** |
| **3** | **c.367A>G** | **p.M123V** |
| **3** | **c.400G>T** | **p.G134*** |
| **3** | **c.413+2T>G** | **Splicing acceptor** |
| **4** | **c.518T>G** | **p.L173W** |
| **5** | **c.637_641delAGTAA** | **p.S213*** |
| **5** | **c.655T>C** | **p.Y219H** |
| **6** | **c.677T>A** | **p.M226K** |
| **6** | **c.694C>T** | **p.R232*** |
| **6** | **c.724delG** | **p.D242Ifs*35** |
| **6** | **c.732G>A** | **p.W244*** |
| **6** | **c.761A>G** | **p.H254R** |
| **6** | **c.772C>T** | **p.L258F** |
| **6** | **c.791delT** | **Deletion** |
| **7** | **c.802-8_806dell3** | **Exon 7 deletion** |
| **7** | **c.802-8_810dell7insGC** | **Exon 7 deletion** |
| **7** | **c.810delT** | **p.(N271Rfs*34)** |
| **7** | **c.838G>T** | **p.E280*** |
| **7** | **c.958C>T** | **p.R320*** |
| **7** | **c.971A>T** | **p.D324V** |
| **7** | **C.974C>T** | **p.T325I** |
| **IVS7** | **c.985+3A>G** | **Unknown** |
| **8** | **c.992A>C** | **p.H331P** |
| **8** | **C.998C >A** | **p.T333K** |
| **8** | **c.1020G>A** | **p.W340*** |
| **8** | **c. l021T>C** | **p.S341P** |
| **8** | **c.1027 T>G** | **p.Y343D** |
| **8** | **c.1062dupA** | **p.V355Sfs*4** |
| **IVS8** | **c. 1091-2A>G** | **Exon 9 deletion** |
| **9** | **c.1157 A>C** | **p.K386T** |
| **9** | **c.1168C>T** | **p.R390C** |
| **9** | **c.1169G>A** | **p.R390H** |
| **9** | **c.11780T** | **p.P393L** |
| **9** | **c.11870T** | **p.P396L** |
| **9** | **c.1198C>T** | **p.R400C** |
| **9** | **c.11990A** | **p.R400H** |
| **9** | **c.l219G>T** | **p.E407*** |
| **9** | **c.1225+1 G>A** | **p.(G364_V408del)** |
| **10** | **c.1226-6_1235dell6** | **Exon 10 deletion** |
| **10** | **c.l328G>A** | **p.R443Q** |
| **10** | **c.13480T** | **p.Q450*** |
| **10** | **c. l355G>A** | **p.R452H** |
| **10** | **c. l372G>A** | **p.V458M** |
| **10** | **c. l393A>G** | **p.R465G** |
| **10** | **c.1396 A>G** | **p.N466D** |
| **10** | **c. l399T>C** | **p.C467R** |
| 10 | c.1441delT | p.(S481Rfs*4) |
| 10 | c.1445C>T | p.S482* |
| 11 | c. 1523G>A | p.R508H |
| 11 | c.1526C>T | p.P509L |

The CYP4V2 gene mutants shown in Table 1 are available from the database https://www.ncbi.nlm.nih.gov/clinvar/?term=CYP4V2[gene].

Without intention to be limited by any theory, the following Examples are only intended to illustrate the nucleic acid molecules, preparation methods, uses, etc. in the present application, and are not intended to limit the scope of the claimed invention. The Examples do not include detailed descriptions of traditional methods, such as methods for constructing vectors and plasmids, methods for inserting the genes encoding proteins into such vectors and plasmids, or methods for introducing plasmids into host cells. Such methods are well known to those of ordinary skill in the art, and are described in numerous publications, including Sambrook, J., Fritsch, E. F. and Maniais, T. (1989) Molecular Cloning: A Laboratory Manual, 2nd edition, Cold spring Harbor Laboratory Press. The unspecified chemical reagents can be purchased through conventional commercial channels.

### Example

### Example 1 Validation of promoter strength by CYP4V2 expression

(1) The full-length CYP4V2 promoter sequence is 2000 bp upstream of the coding region of human genome CYP4V2 (HGNC: 23198), and was synthesized by Genewiz (Suzhou).
(2) The primer sequences for promoters CYP4V2-Pf, CYP4V2-P1, CYP4V2-P2, CYP4V2-P3, CYP4V2-P4, CYP4V2-P5, and CYP4V2-P6 with different lengths were designed for PCR amplification using the nucleic acid molecule obtained in step (1) as the template, and the amplification products were recovered by gel electrophoresis. The primer sequences are shown in SEQ ID NOs: 30-37.

**The PCR reaction system (50µl) was as follows:**

| | |
|---|---|
| H₂O (Invitrogen, 10977015) | 20µl |
| Primer F (10 pmol/µl) | 2µl |
| Primer R (10 pmol/µl) | 2µl |
| primeSTAR MAX (Takrara, R045R) | 25µl |
| CYP4V2-pro-2000bp | 5ng |

**Table 2 PCR reaction conditions**

| **Temperature** | **Time** | **Cycle number** |
|---|---|---|
| **95°C** | **5min** | **1** |
| **98°C** | **10s** | **30** |
| **55-60°C** | **15s** | |
| **72°C** | **20s** | |
| **72°C** | **10min** | **1** |
| **16°C** | **∞** | **1** |

(3) The pAV-CAG-CYP4V2-P2A-EGFP vector (purchased from Shandong Weizhen Biotechnology Co., Ltd.) was amplified from both ends of CAG promoter, and the amplification products were recovered by gel electrophoresis. The primer sequences are shown in SEQ ID NOs: 38-39. The PCR reaction system (50µl) was as follows:

| | |
|---|---|
| H₂O (Invitrogen, 10977015) | 20µl |
| Primer F (10 pmol/µl) | 2µl |
| Primer R (10 pmol/µl) | 2µl |
| primeSTAR MAX (Takrara, R045R) | 25µl |
| pAV-CAG-CYP4V2-P2A-EGFP | 5ng |

**Table 3 PCR reaction conditions**

| **Temperature** | **Time** | **Cycle number** |
|---|---|---|
| **95°C** | **5min** | **1** |
| **98°C** | **10s** | **30** |
| **55-60°C** | **15s** | |
| **72°C** | **20s** | |
| **72°C** | **10min** | **1** |
| **16°C** | **∞** | **1** |

(4) Different CYP4V2 promoter sequences (set forth in SEQ ID NOs: 6-12) obtained in step (2) were homologously ligated into the linearized pAV-CAG-CYP4V2-P2A-EGFP vector obtained in step (3), to replace the CAG promoter in the original vector.
The ligation system (operated on ice) was as follows:

| | |
|---|---|
| Linearized pAV-CAG-CYP4V2-P2A-EGFP vector | 1-2 µl |
| CYP4V2-Pf/P1/P2/P3/P4/P5/P6 promoter fragment | 2-4 µl |
| Lightening Cloning Master Mix (Biodragon, BDIT0014-20) | 5 µl |
| ddH₂O | x µl |
| Total | 10 µl |

After placing in a 50°C water bath for 40 min, 5 µl of the reaction solution was used for the transformation of transStbl3 *E. coli* competent cells. After shaking culture and sequencing, the plasmid was extracted using Plasmid Midi Kit (Omega, D6915-04).
(5) 293T cells (ATCC, CRL-3216) were plated onto a 35 mm dish on the first day, and reached to about 70% confluence on the second day. The transfection system was formulated as follows: to 100 µl of serum-free DMEM, 2 µg of the plasmid obtained in step (4) and 3 µl of PEI were added respectively and mixed well, to stand for 20 min; the obtained transfection system was added to the cell medium, shaken well, and placed in a CO₂ incubator; and the medium was replaced after 6 h or overnight.
(6) After 48 hours, the cells were lysed with RIPA lysis buffer (Beijing Applygen C1053-100), for running in the gel. The expression of CYP4V2 was detected by Western blot. The following antibodies were used: anti-CYP4V2 (Atlas, HPA029122), anti-actin (Abclonal, AC026), goat-anti-rabbit (Abclonal, AS014).

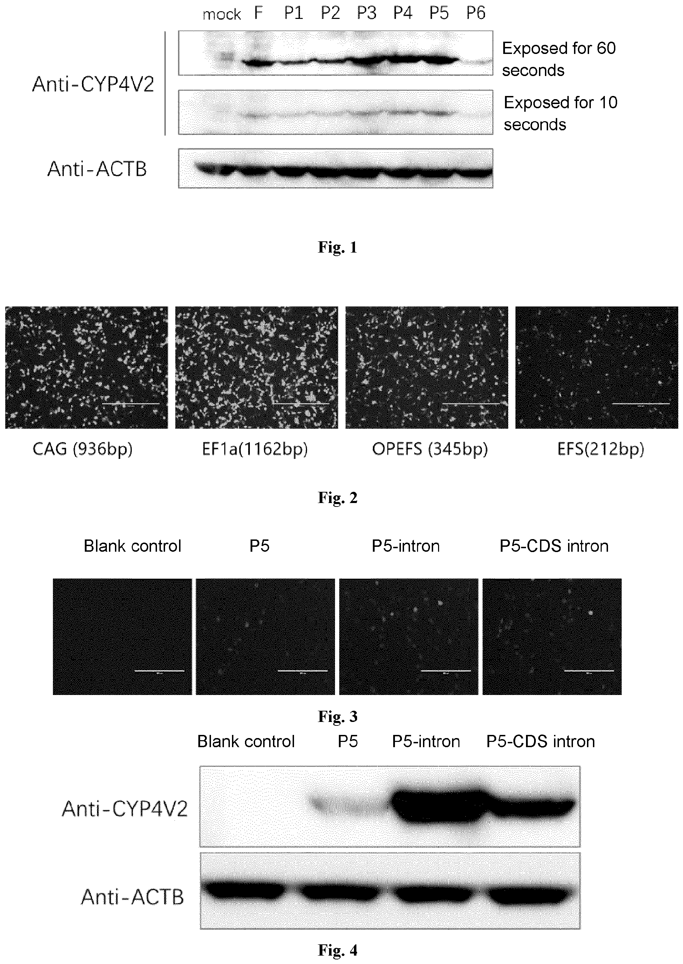

The results were shown in Fig. 1. All of the CYP4V2-PF promoter, CYP4V2-P1 promoter, CYP4V2-P2 promoter, CYP4V2-P3 promoter, CYP4V2-P4 promoter, CYP4V2-P5 promoter, and CYP4V2-P6 promoter could achieve the expression of CYP4V2, wherein the CYP4V2-PF promoter, CYP4V2-P3 promoter, CYP4V2-P4 promoter, and CYP4V2-P5 promoter had better expression results, while the CYP4V2-P5 sequence was shorter and more advantageous.

### Example 2. Validation of promoter strength using EGFP as reporter gene

(1) The promoters EF1a, EFS, and OPEFS were amplified by PCR. The Ef1a primer sequence is set forth in SEQ ID NOs: 40-41, and the EFS primer sequence is set forth in SEQ ID NOs: 42-43. The OPEFS promoter was based on the universal promoter EFS, and through designing primers, EFS was engineered via two rounds of PCR, to obtain OPEFS. The primers used in the first round of PCR reaction were EFS forward primer (set forth in SEQ ID NO: 42) and OPEFS-overlapR1 (set forth in SEQ ID NO: 44), and the primers used in the second round of PCR reaction were EFS forward primer (set forth in SEQ ID NO: 42) and OPEFS-overlapR2 (set forth in SEQ ID NO: 45). The amplification products were recovered by gel electrophoresis. The PCR reaction system (50µl) was as follows:

| | |
|---|---|
| H2O (Invitrogen, 10977015) | 20µl |
| Primer F (10 pmol/µl) | 2µl |
| Primer R (10 pmol/µl) | 2µl |
| primeSTAR MAX (Takrara, R045R) | 25µl |
| pAV-CAG-CYP4V2-P2A-EGFP | 5ng |

**Table 4 PCR reaction conditions**

| **Temperature** | **Time** | **Cycle number** |
|---|---|---|
| **95°C** | **5min** | **1** |
| **98°C** | **10s** | **30** |
| **55-60°C** | **15s** | |
| **72°C** | **20s** | |
| **72°C** | **10min** | **1** |
| **16°C** | **∞** | **1** |

(2) The amplification products obtained in step (1) (nucleic acid molecules for promoters OPEFS, EFS, and EF1a, with sequences set forth in SEQ ID NOs: 1-3) were ligated by homologous recombination into the linearized pAV-CAG-CYP4V2-P2A-EGFP obtained in accordance with step (3) of Example 1, to obtain pAV-EF1a-CYP4V2-P2A-EGFP, pAV-EFS-CYP4V2-P2A-EGFP, and pAV-OPEFS-CYP4V2-P2A-EGFP. The ligation system (operated on ice) was as follows:

| | |
|---|---|
| Linearized pAV-CAG-CYP4V2-P2A-EGFP vector | 1-2 µl |
| EF1a, EFS, OPEFS fragments | 2-4 µl |
| Lightening Cloning Master Mix (Biodragon, BDIT0014-20) | 5 µl |
| ddH₂O | x µl |
| Total | 10 µl |

After placing in a 50°C water bath for 40 min, 5 µl of the reaction solution was used for the transformation of transStbl3 *E. coli* competent cells. After shaking culture and sequencing, the plasmid was extracted using Plasmid Midi Kit (Omega, D6915-04).
(3) In accordance with the procedure in step (5) of Example 1, by using PEI (Polysciences 24765-1), the plasmids pAV-CAG-CYP4V2-P2A-EGFP, pAV-EF1a-CYP4V2-P2A-EGFP, pAV-EFS-CYP4V2-P2A-EGFP, and pAV-OPEFS-CYP4V2-P2A-EGFP were respectively transfected into 293T cells (ATCC, CRL-3216) plated onto 6-well plates in advance, with a cell density of about 70%.
(4) The fluorescence signal was observed by fluorescence microscope (Life AMF4305) after 24 hours.

The results were shown in Fig. 2. Compared with EFS, CAG, EF1a, and OPEFS all had better expression results, wherein the OPEFS sequence was shorter and more advantageous.

### Example 3 Validation of the enhancement effect of intron

I. Cloning of an intron into the 5' non-coding region of the gene.
(1) Cloning of P5-intron. Based on the CYP4V2-P5 promoter sequence and human β-globin intron, primers were designed, and P5-intron was obtained by engineering through overlapping PCR. 1) With the CYP4V2-P5 promoter sequence as the template, the CYP4V2-P5 forward primer (CYP4V2-P5F, set forth in SEQ ID NO: 46) and the CYP4V2-P5 (in) reverse primer (set forth in SEQ ID NO: 47) were employed for PCR amplification; 2) With the human β-globin intron as the template, the β-globin intron (c) (set forth in SEQ ID NO: 48) and the β-globin intron reverse primer (set forth in SEQ ID NO: 49) were employed for PCR amplification; and 3) The amplification products were recovered, respectively. With the target products obtained in 1) and 2) as common templates, the CYP4V2-P5 forward primer (CYP4V2-P5F, set forth in SEQ ID NO: 46) and the β-globin intron reverse primer (set forth in SEQ ID NO: 49) were employed for PCR amplification. The PCR amplification was performed in accordance with the system and condition in step (1) of Example 2. The target product P5-intron obtained by amplification was recovered.
(2) The P5-intron sequence obtained by amplification was sequenced, and then ligated by homologous recombination into the linearized pAV-CAG-CYP4V2-P2A-EGFP that was obtained in accordance with step (3) of Example 1, to produce pAV-p5-intron-CYP4V2-P2A-EGFP. The ligation system (operated on ice) was as follows:

| | |
|---|---|
| Linearized pAV-CAG-CYP4V2-P2A-EGFP vector | 1-2 µl |
| P5-intron fragment | 2-4 µl |
| Lightening Cloning Master Mix (Biodragon, BDIT0014-20) | 5 µl |
| ddH₂O | x µl |
| Total | 10 µl |

(3) After placing in a 50°C water bath for 40 min, 5 µl of the reaction solution was used for the transformation of transtb 13 *E. coli* competent cells. After shaking culture and sequencing, the plasmid was extracted using Plasmid Midi Kit (Omega, D6915-04).
II. Cloning of the intron into the coding region of the gene.
(1) Cloning of CYP4V2-CDSintron. In this fragment, the intron was located between the first and second exons of CYP4V2. Based on the CDS sequence of CYP4V2 and the human β-globin intron, primers were designed, and CDSintron was obtained by engineering through overlapping PCR.
1) With the CDS sequence of CYP4V2 as the template, the primer pairs CYP4V2-Fpr/CYP4V2-E1-R (sequences set forth in SEQ ID NOs: 50 and 51, respectively) and CYP4V2-E2-F/CYP4V2-R (sequences set forth in SEQ ID NOs: 52 and 53, respectively) were employed for PCR amplification, to obtain PCR products CYP4V2-E1 and CYP4V2-E2∼11, respectively;
2) With the β-globin intron as the template, the primer pair Intron-F/Intron-R (sequences set forth in SEQ ID NOs: 54 and 55, respectively) was employed for PCR amplification;
3) The amplification products were recovered, respectively. With the target product CYP4V2-E1 obtained in 1) and the target product obtained in step 2) as common templates, the CYP4V2-CDS forward primer CYP4V2-Fpr (sequence set forth in SEQ ID NO: 50) and the β-globin intron reverse primer Intron-R (sequence set forth in SEQ ID NO: 55) were employed for PCR amplification. The PCR amplification was performed in accordance with the system and condition in step (1) of Example 2. The target product CYP4V2-E1-intron obtained by amplification was recovered.
4) With the target product CYP4V2-E1-intron obtained in 3) and the target products CYP4V2-E2∼11 obtained in 1) as common templates, the CYP4V2-CDS forward primer CYP4V2-Fpr and the CYP4V2-CDS reverse primer CYP4V2-R were employed for PCR amplification. The PCR amplification was performed in accordance with the system and condition in step (1) of Example 2.
(2) The CDSintron sequence obtained by amplification was sequenced.
(3) After a restriction enzyme digest of pAV-p5-CYP4V2-P2A-EGFP at 37°C for 2h, a linearized vector was obtained and recovered by gel electrophoresis. The restriction enzyme system was as follows:

| | |
|---|---|
| pAV-p5-CYP4V2-P2A-EGFP | 2µg |
| EcoRI (NEB, R3101S) | 2µl |
| MluI (NEB, R3198S) | 2µl |
| Cutsmart Buffer (NEB) | 4µl |
| H₂O (Invitrogen, 10977015) | to 40µl |

(4) CYP4V2-CDSintron was ligated by homologous recombination into the linearized pAV-p5-CYP4V2-P2A-EGFP, to obtain pAV-p5-CDSintron-CYP4V2-P2A-EGFP. The ligation system (operated on ice) was as follows:

| | |
|---|---|
| Linearized pAV-p5-CYP4V2-P2A-EGFP vector | 1-2 µl |
| CYP4V2-CDS intron fragment | 2-4 µl |
| Lightening Cloning Master Mix (Biodragon, BDIT0014-20) | 5 µl |
| ddH₂O | x µl |
| Total | 10 µl |

(5) After placing in a 50°C water bath for 40 min, 5 µl of the reaction solution was used for the transformation of transStbl3 *E. coli* competent cells. After shaking culture and sequencing, the plasmid was extracted using Plasmid Midi Kit (Omega, D6915-04).
III. Validation of the enhancement effect of intron located in the 5' non-coding region and the coding region of the gene
(1) In accordance with the procedure in step (5) of Example 1, by using PEI (Polysciences 24765-1), pAV-p5-CYP4V2-P2A-EGFP, pAV-p5-intron-CYP4V2-P2A-EGFP, and pAV-p5-CDSintron-CYP4V2-P2A-EGFP were transfected into 293T cells (ATCC, CRL-3216) plated onto 6-well plates in advance, with a cell density of about 70%.
(2) The fluorescence signal was observed by fluorescence microscope (Life AMF4305) after 24 hours.
(3) After 48 hours, the cells were lysed with RIPA lysis buffer (Beijing Applygen C1053-100), for running in the gel. The expression of CYP4V2 was detected by Western blot. The following antibodies were used: anti-CYP4V2 (Atlas, HPA029122), anti-actin (Abclonal, AC026), goat-anti-rabbit (Abclonal, AS014).

The results were shown in Fig. 3 and Fig. 4. When the human β-globin intron was inserted into the 5' non-coding region and the coding region of the gene, the expression effect was enhanced.

### Example 4 Selection of PolyA signal site

(1) SV40 in the pAV-OPEFS-CYP4V2-P2A-EGFP vector obtained in Example 2 was replaced by BGH, WPRE, WPRE-SV40, and WPRE-BGH (sequences set forth in SEQ ID NOs: 19-21), respectively, to obtain pAV-OPEFS-CYP4V2-P2A-EGFP-BGH, pAV-OPEFS-CYP4V2-P2A-EGFP-WPRE, pAV-OPEFS-CYP4V2-P2A-EGFP-WPRE-SV40, and pAV-OPEFS-CYP4V2-P2A-EGFP-WPRE-BGH. The gene synthesis and subcloning here were accomplished by Beijing Tsingke Biotechnology.
(2) The vector obtained in step (1) was identified by sequencing, and then the plasmid was extracted using Plasmid Midi Kit (Omega, D6915-04).
(3) In accordance with the procedure in step (5) of Example 1, by using PEI (Polysciences 24765-1), the plasmids obtained in step (2) of this Example were respectively transfected into 293T cells (ATCC, CRL-3216) plated onto 6-well plates in advance, with a cell density of about 70%.
(4) The fluorescence signal was observed by fluorescence microscope (Life AMF4305) after 24 hours.
(5) After 48 hours, the cells were lysed with RIPA lysis buffer (Beijing Applygen C1053-100), for running in the gel. The expression of CYP4V2 was detected by Western blot. The following antibodies were used: anti-CYP4V2 (Atlas, HPA029122), anti-actin (Abclonal, AC026), goat-anti-rabbit (Abclonal, AS014).

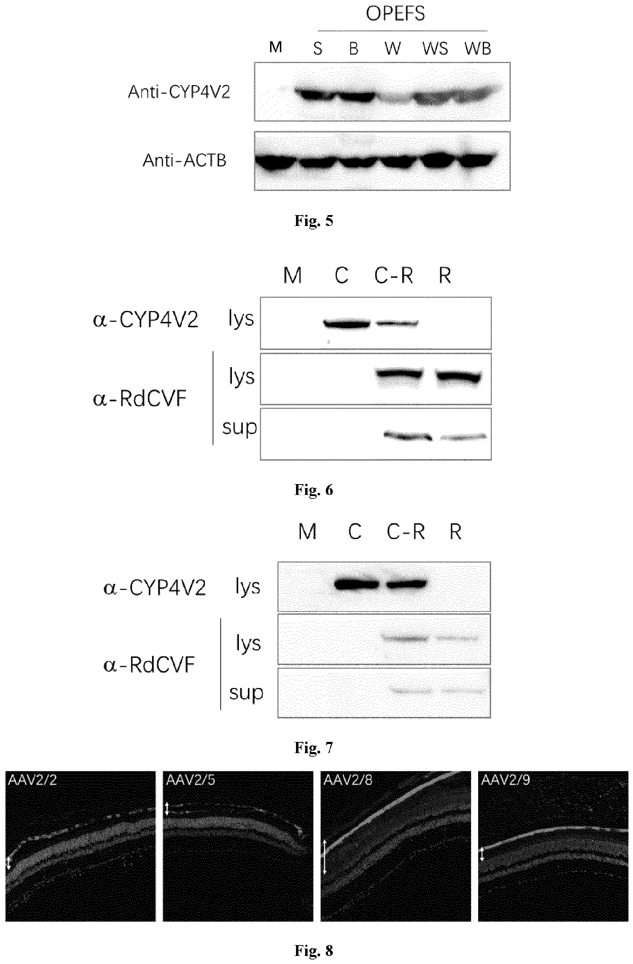

The results were shown in Fig. 5, where M (mock) represents the blank control, S represents SV40, B represents BGH, W represents WPRE, WS represents WPRE-SV40, and WB represents WPRE-BGH. Compared with the control group, the expression of CYP4V2 can be detected in each experimental group.

### Example 5 Construction of expression vector

(1) Construction of pAV-OPEFS-CYP4V2-BGH, pAV-OPEFS-RdCVF-BGH, and pAV-OPEFS-CYP4V2-P2A-RdCVF-BGH (kanamycin-resistant). The ampicillin resistant gene in pAV-OPEFS-CYP4V2-P2A-EGFP-BGH was replaced by the kanamycin-resistant gene. The vector engineering was accomplished by Genewiz (Suzhou); and the related gene synthesis and vector engineering (kanamycin-resistant) for pAV-OPEFS-CYP4V2-BGH (expression sequence set forth in SEQ ID NO: 90), pAV-OPEFS-RdCVF-BGH (expression sequence set forth in SEQ ID NO: 96), and pAV-OPEFS-CYP4V2-P2A-RdCVF-BGH (expression sequence set forth in SEQ ID No: 106) were accomplished by Genewiz (Suzhou).
(2) Construction of pAV-p5-intron-CYP4V2-BGH (expression sequence set forth in SEQ ID NO: 93), pAV-p5-intron-RdCVF-BGH (expression sequence set forth in SEQ ID NO: 99), and pAV-p5-intron-CYP4V2-P2A-RdCVF-BGH (expression sequence set forth in SEQ ID NO: 113) (kanamycin-resistant). After the restriction enzyme digestion of pAV-OPEFS-CYP4V2-BGH, pAV-OPEFS-RdCVF-BGH, and pAV-OPEFS-CYP4V2-P2A-RdCVF-BGH (kanamycin-resistant) at 37°C for 2h, linearized vectors were obtained and recovered by gel electrophoresis. The restriction system was as follows:

| | |
|---|---|
| Vector plasmid | 2µg |
| EcoRI (Takara D1040A) | 2µl |
| BamHI(Takara D1010A) | 2µl |

| | |
|---|---|
| 10×K Buffer(Takara) | 4µl |
| H₂O (Invitrogen, 10977015) | to 40µl |

(3) The P5-intron fragment was obtained by PCR amplification from pAV-p5-intron-CYP4V2-P2A-EGFP, and recovered by gel electrophoresis. The forward primer set forth in SEQ ID NO: 46 and the reverse primer set forth in SEQ ID NO: 55 were used.
(4) The enzyme digested recovery vector was ligated to the linear fragment for recombination with the vector. The ligation system (operated on ice) was as follows:

| | |
|---|---|
| Enzyme digested vector obtained in step (2) | 1-2 µl |
| P5-intron fragment | 2-4 µl |
| Lightening Cloning Master Mix (Biodragon, BDIT0014-20) | 5 µl |
| ddH₂O | x µl |
| Total | 10 µl |

After placing in a 50°C water bath for 40 min, 5 µl of the reaction solution was used for the transformation of transStbl3 *E. coli* competent cells. After shaking culture and sequencing, the plasmid was extracted using Plasmid Midi Kit (Omega, D6915-04).

### Example 6 Expression detection of expression vector in 293T cells

(1) In accordance with the procedure in step (5) of Example 1, by using PEI (Polysciences 24765-1), the plasmids pAV-OPEFS-CYP4V2-BGH, pAV-OPEFS-RdCVF-BGH, and pAV-OPEFS-CYP4V2-P2A-RdCVF-BGH obtained in step (a) of Example 5 were transfected into 293T cells (ATCC, CRL-3216) plated onto 6-well plates in advance, with a cell density of about 70%.
(2) After 48 hours, the cell medium supernatant (sup) was collected, and the cells were washed with PBS and then lysed with RIPA lysis buffer (Beijing Applygen C1053-100) (lys). The expression of CYP4V2 and/or RdCVF in the supernatant or cell lysate (lys) in each group was detected by Western blot. The following antibodies were used: anti-CYP4V2 (Atlas, HPA029122), anti-actin (Abclonal, AC026), goat-anti-rabbit (Abclonal, AS014), RdCVF Antibody (TXNL6 Antibody, H00115861-D01P).

The results were shown in Fig. 6, where M (mock) represents the blank control, C represents the OPEFS-CYP4V2 group, C-R represents the OPEFS-CYP4V2-P2A-RdCVF group, and R represents the OPEFS-RdCVF group. For the cells in the blank control group (M group), the expression of CYP4V2 or RdCVF was not detected in either the lysate (lys) or the cell medium supernatant (sup); for the cell transfected with pAV-OPEFS-CYP4V2-BGH, the expression of CYP4V2 was detected in the lysate; for the cells transfected with pAV-OPEFS-RdCVF-BGH, the expression of RdCVF was detected in both the supernatant and the lysate; and for the cells transfected with pAV-OPEFS-CYP4V2-P2A-RdCVF-BGH, the expression of CYP4V2 was detected in the lysate, and the expression of RdCVF was detected in both the lysate and the supernatant.

### Example 7 Expression detection of expression vector in ARPE-19 cells

(1) In accordance with the procedure in step (5) of Example 1, by using PEI (Polysciences 24765-1), the plasmids pAV-OPEFS-CYP4V2-BGH, pAV-OPEFS-RdCVF-BGH, and pAV-OPEFS-CYP4V2-P2A-RdCVF-BGH obtained in step (a) of Example 5 were transfected into ARPE-19 cells (ATCC, CRL-2302) plated onto 6-well plates in advance, with a cell density of about 70%.
(2) After 48 hours, the cell medium supernatant (sup) was collected, and the cells were washed with PBS and then lysed with RIPA lysis buffer (Beijing Applygen C1053-100) (lys). The expression of CYP4V2 and/or RdCVF in the supernatant or cell lysate (lys) in each group was detected by Western blot. The following antibodies were used: anti-CYP4V2 (Atlas, HPA029122), anti-actin (Abclonal, AC026), goat-anti-rabbit (Abclonal, AS014), TXNL6 Antibody (H00115861-D01P).

The results were shown in Fig. 7, where M (mock) represents the blank control, C represents the OPEFS-CYP4V2 group, C-R represents the OPEFS-CYP4V2-P2A-RdCVF group, and R represents the OPEFS-RdCVF group. For the cells in the blank control group (mock group), the expression of CYP4V2 or RdCVF was not detected in either the lysate or the cell medium supernatant; for the cell transfected with pAV-OPEFS-CYP4V2-BGH, the expression of CYP4V2 was detected in the lysate; for the cells transfected with pAV-OPEFS-RdCVF-BGH, the expression of RdCVF was detected in both the supernatant and the lysate; and for the cells transfected with pAV-OPEFS-CYP4V2-P2A-RdCVF-BGH, the expression of CYP4V2 was detected in the lysate, and the expression of RdCVF was detected in both the lysate and the supernatant.

### Example 8 Viral vector

I. Selection of AAV serotype: the viruses of AAV2/2, AAV2/5, AAV2/8, and AAV2/9 serotypes (purchased from Shandong Weizhen Biotechnology Co., Ltd.) packaging GFP reporter gene were injected into the subretinal spaces of wild-type mice. After 1 month, the retinal histomorphology was observed by sectioning. The results were shown in Fig. 8, wherein the double arrows indicated the expression range of the EGFP reporter gene. The AAV2/8 serotype with a good preference for the retina was selected.
   Construction of AAV2/8 vector:
   (1) The engineering was based on pAAV-RC5-Amp (purchased from Beijing XMJ Scientific Co., Ltd., vector sequence set forth in SEQ ID NO: 133). The pAAV-RC5 sequence except Amp was amplified by PCR, using the primers RC5-F/RC5-R set forth in SEQ ID NOs: 56-57.
   (2) The Kana part was amplified from pEGFP-N1 by PCR, using the primers Kana-F/Kana-R set forth in SEQ ID NOs: 58-59.
   (3) The two DNA fragments obtained by PCR as above were subjected to the homologous recombination to obtain pAAV-RC5-Kana.
   (4) Through the engineering based on pAAV-RC5-Kana, the pAAV-Kana sequence except RC5-cap was amplified by PCR, using the primers RC5-Kana-F/RC5-Kana-R set forth in SEQ ID NOs: 60-61.
   (5) The RC8-cap sequence by gene synthesis (Beijing Tsingke Biotechnology, sequence set forth in SEQ ID NO: 134) and the pAAV-Kana linearized vector obtained in step (4) were subjected to the homologous recombination to obtain pAAV-RC8-Kana (i.e., AAV2/8) (vector sequence set forth in SEQ ID NO: 135).
II. AAV-helper selection: A universal helper plasmid AAV-helper was selected to change the resistance from ampicillin to kanamycin. The specific building process was as follows:
   1) The engineering was based on pAAV-helper-Amp (purchased from Beijing XMJ Scientific Co., Ltd., vector sequence set forth in SEQ ID NO: 136). The pAAV-helper sequence except Amp was amplified by PCR, using the primers RC5-F/RC5-R set forth in SEQ ID NOs: 56-57.
   2) The Kana part was amplified from pEGFP-N1 by PCR, using the primers Kana-F/Kana-R set forth in SEQ ID NOs: 58-59.
   3) The two DNA fragments obtained by PCR as above were subjected to the homologous recombination to obtain pAAV-helper-Kana (i.e., AAV-helper) (vector sequence set forth in SEQ ID NO: 137).

### Example 9 Virus packaging and purification

I. Virus packaging
(1) Day 0: Cell seeding (seeding number: 1×10⁷): 293T cells were seeded in a 15 cm dish.
(2) Day 1: When 293T cells reached 80%-90% confluence, the medium was replaced with 20 ml of complete medium (Gibco, C11965500BT) containing 10% serum (Shanghai ExCell, FSP500) for plasmid transfection. The specific transfection system was shown in Table 5.

**Table 5 Transfection system**

| System components | per 15 cm dish |
|---|---|
| Expression vector | 15µg |
| AAV-helper | 15µg |
| AAV2/8 | 15µg |
| Plasmid mix | 45 µg |
| Serum-free DMEM (Gibco, C11965500BT) | 2000µl |
| After adding plasmid, mixing well, then adding PEI | |
| PEI (Polysciences 24765-1) | 135µl |
| After adding PEI, mixing by hand (do not vortex), and incubating at room temperature for 20 min. | |

The expression vector in Table 5 was the expression vector constructed in accordance with the procedure of Example 5. (3) Day 2: after transfecting for 12-18 hours, the medium was replaced with 30 ml of fresh complete medium.
(4) Day 3: after transfecting for 48 hours, the medium was replaced with 30 ml of fresh complete medium.
(5) Day 4: 72h after transfection, 293T cells were trypsinized according to the conventional method, collected into a 50 ml centrifuge tube, washed twice with PBS, centrifuged at 1200 rpm for 5 min, and frozen at -80°C refrigerator after removing PBS.
II. Virus purification
1) The AAV293T cells obtained in step (5) of this Example were thawed at 15°C-25°C.
2) The cells in each 15 cm dish were resuspended with 2 ml of cell lysis buffer (150 mM NaCl, 50 mM Tris, pH 8.5) (the cells in every three 15 cm-dishes were collected in a 50 ml centrifuge tube, i.e., 6 ml of cell lysate).
3) Half volume (3 ml, relative to the cell lysate) of chloroform was added to the centrifuge tube, the tube cap was tighten, and the tube was horizontally placed on a shaker at 37°C and shaken at 250 rpm for 30 min.
4) A quarter volume (1.5 ml, relative to the aqueous phase) of 5M sodium chloride was added and mixed well, transferred to a high-speed centrifuge tube, and centrifuged at 11,000 rpm for 25 min.
5) The upper aqueous phase was taken. The part at the junction that was difficultly pipetted was transferred to a 1.5 ml centrifuge tube, and centrifuged at 12,000 rpm for 30 s. The supernatants were combined. The nuclease (Benzonase) was added to the upper aqueous phase to a final concentration of 50 U/ml, the sodium deoxycholate was added to a final concentration of 0.4%, and these were added to the following final concentrations: 10 mM of MgCl₂, 0.5 mM of CaCl₂, 5 IU/ml of Turbo DNase I, 25 ug/ml of RNaseA (stock solution concentration: 10 mg/ml).
6) 37°C water bath for 30 min.
7) A quarter volume (2 ml, relative to the aqueous phase) of 50% PEG8000 was added, mixed well by shaking, placed in ice for 1 hour, and centrifuged at 11,000 rpm for 25 min.
8) The supernatant was aspirated and discarded, and the remaining was centrifuged again for 1 min to remove the residual supernatant.
9) PBS was added according to the final dissolving volume requirement, suspended by pipetting, and transferred to a 1.5 ml centrifuge tube, wherein during the transferring it was pipetted at a small volume (<200µl) to avoid the loss of viruses due to sediment and adhesion.
10) 500 µl of chloroform was added, shaken, and centrifuged at 12,000 rpm for 20 min.
11) The supernatant was pipetted under sterile conditions, and stored in aliquots at -80°C.

### Example 10 Determination of virus titer

I. Formulation of pAAV-EGFP standard
1. Gradients for the standard: 1×10⁸ copies/5µl, 1×10⁷ copies/5µl, 1×10⁶ copies/5µl, 1×10⁵ copies/5µl, 1×10⁴ copies/5µl, 1×10³ copies/µl, and 1×10² copies/5µl.
2. Dilution steps:
   a. The stock solution was ten-fold diluted and quantified by Nano, and subsequent dilutions were based on this quantitative result.
   b. The quantified sample was diluted stepwise to 1 ng/µl of plasmid.
   c. The sample (1 ng/µl) was diluted to 1×10⁸ copies/µl.
   d. The diluted solution (1×10⁸ copies/µl) was five-fold diluted to 1×10⁸ copies/5µl.
   e. The solution was ten-fold diluted sequentially to 1×10² copies/5µl.
II. Treatment and dilution of sample
1. 17 µl Dnase1 + 1 ml 1×buffer (TransGen Gd-201-01) were formulated into a 50U/ml buffer.
2. The sample was diluted by adding 95 µl of buffer formulated in step 1 to 5 µl of the sample to be tested as obtained in step 11) of Example 9 (10-fold dilution).
3. The sample was mixed well (vortexing for 5 s), and placed in the PCR instrument at 37°C for 30 min; at 75°C for 15 min.
4. The reaction solution in step 3 was shaken and mixed well, and 50 µl of the solution was added to 450 µl of nuclease-free water to obtain the first dilution gradient (200-fold dilution).
5. 50 µl of the first gradient reaction solution mixed in step 4 was added to 450 µl of nuclease-free water to obtain the second dilution gradient (2000-fold dilution).
6. 50 µl of the first gradient reaction solution mixed in step 5 was added to 450 µl of nuclease-free water to obtain the third dilution gradient (2000-fold dilution).
III. Fluorescence quantitative PCR

**Table 6 Fluorescence quantitative PCR amplification system:**

| | |
|---|---|
| **SYBR Master Mix 2 × (ABI, 4472908)** | **10 µl** |
| **10uM ITR-F (set forth in SEQ ID NO:154)** | **1 µl** |
| **10 uM ITR-R (set forth in SEQ ID NO:155)** | **1 µl** |
| **Sample to be tested** | **5 µl** |
| **H2O** | **3 µl** |
| **Total** | **20µl** |

**Table 7 Fluorescence quantitative PCR amplification conditions (ABI 7500 real-time fluorescent quantitative PCR instrument):**

| **1 cycle** | **1 cycle** | **40 cycles** | |
|---|---|---|---|
| **50°C** | **95°C** | **95°C** | **60°C** |
| **2 min** | **2 min** | **15s** | **31s** |

**Table 8 Quantitative results for virus titer**

| Name | Sample Name | Qty | Qty × dilution factor | Average concentration vg/ml |
|---|---|---|---|---|
| AAV-OPEFS-EGFP | E200 | 2.91E+08 | 1.16E+10 | 1.00E+ 13 |
| | E2000 | 2.47E+07 | 9.89E+09 | |
| | E20000 | 2.14E+06 | 8.56E+09 | |
| AAV-OPEFS-CYP4V2 | C200 | 8.75E+08 | 3.50E+10 | 3.06E+13 |
| | C2000 | 1.35E+08 | 5.42E+10 | |
| | C20000 | 6.51E+05 | 2.61E+09 | |

The test results (Table 8) showed that the virus titers for AAV-OPEFS-EGFP and AAV-OPEFS-CYP4V2 were 1.00E+13vg/ml and 3.06E+13vg/ml, respectively, and can be used for subsequent infection experiments. The titers of other viruses used in Examples of the present application were determined according to the process of this Example.

### Example 11 Infection of human iPSC-differentiated RPE cells by different doses of viruses

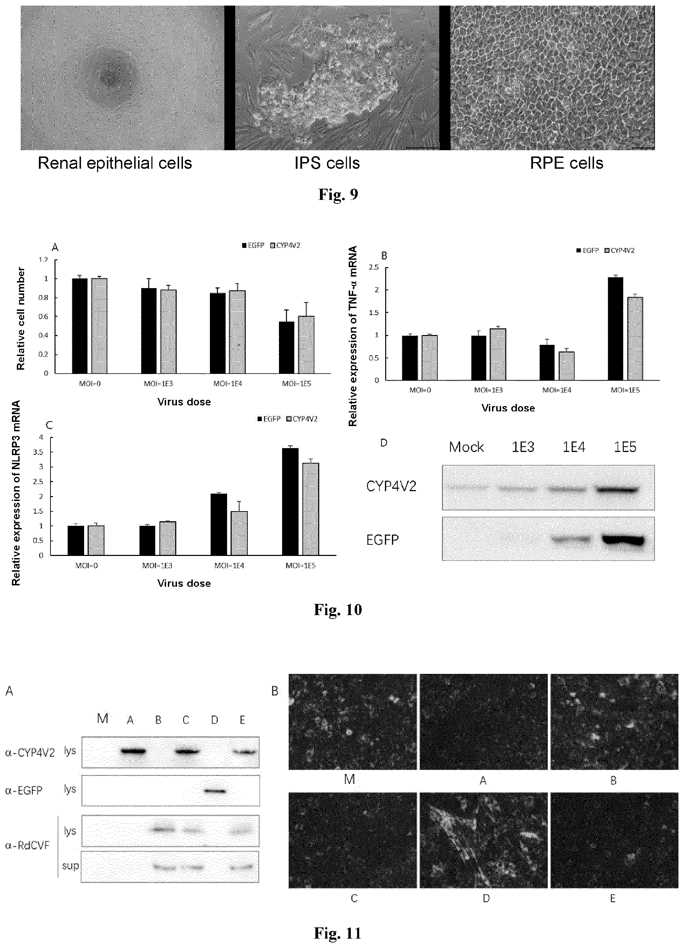

(1) Production of human induced pluripotent stem cell (IPSC). The renal epithelial cells were extracted from urine using Urineasy Urinary Cell Separation Kit (Beijing Cellapy, CA3102500), and cultured and expanded using Urineasy Urinary Cell Expansion Kit (Beijing Cellapy, CA3103200), and the cells with 70-80% confluence at the 3rd to 4th passage were selected for the reprogramming experiment. The reprogramming experiment was performed using the hiPSC Reprogramming Kit (Beijing Cellapy, CA5002002), in accordance with the instructions of kit, to obtain human IPSCs which were used for the next cell differentiation experiment.
(2) Production of RPE cells. In accordance with the process described in the literature (da Cruz, L., et al., (2018), Phase 1 clinical study of an embryonic stem cell-differentiated retinal pigment epithelium patch in age-related macµlar degeneration, Nat Biotechnol 36(4): 328-337.), RPE cells were produced. The human IPSCs (3^{∗}10⁴/cm²) were cultured in 4 ml of TESR-E8 medium (STEMCELL, CAT#05990, #05991) in a T25 flask. The medium was replaced with 6 ml of medium (Gibco, CAT#10829018) containing 20% serum substitute (Gibco, CAT#A3181502) after 5 days, and then replaced with 6 ml of serum-free medium (Gibco, CAT#10829018) after culturing for 2 days. The culture was continued for about 20 weeks, to obtain elliptical and dark dividable cells, i.e., RPE cells. Fig. 9 showed the renal epithelial cells, IPS cells, and RPE cells observed under white light by fluorescence microscopy (Life AMF4305) at 4, 10, and 10 magnifications, respectively.
(3) The same amounts of human iPSC-differentiated RPE cells were infected with packaging viruses containing pAV-OPEFS-EGFP-BGH or pAV-OPEFS-CYP4V2-BGH under dose gradients (MOI=0, 1×10³, 1×10⁴, 1×10⁵).
(4) After 72 hours, the cells were dissociated, and each group was divided into three groups: one group was analyzed for cell death with Cell Titer Glow (Promega, G9241) according to the instructions; one group was detected for CYP4V2 expression by Western blot according to the procedure in step (2) of Example 6; and one group was used for RNA extraction to detect the mRNA levels of inflammatory factors (NLRP3, TNF-α, IFN-γ) by RT-qPCR. RNA was extracted using RNA Extraction Kit (Tiangen, DP430) according to the instructions, and subjected to the reverse transcription to obtain cDNA, using Reverse Transcription Kit (Takara, RR407A) according to the instructions. RT-qPCR was performed in accordance with the procedure in step III of Example 10, and RT-qPCR primer sequences were set forth in SEQ ID NOs: 138-145.

The results were shown in Figs. 10A-10D. With the increase of pAV-OPEFS-EGFP-BGH virus titer, the expression of EGFP, the expression levels of inflammatory factors TNF-α and NLRP3, and the cell mortality rate increased; and with increase of pAV-OPEFS-CYP4V2-BGH virus titer, the expression of CYP4V2, the expression levels of inflammatory factors TNF-α and NLRP3, and the cell mortality rate increased. It was demonstrated that for human iPSC-differentiated RPE cells, AAV viruses with higher titers had a cytotoxicity, and thus a dose of 1×10⁴ or less can be selected for subsequent experiments on human iPSC-differentiated RPE cells.

### Example 12 Effects of infecting iPSC-differentiated RPE cells by various viruses in BCD patients

(1) The virus packaging followed the procedure of Example 7: A: pAV-p5-intron-CYP4V2-BGH (expression sequence set forth in SEQ ID NO: 93); B: pAV-p5-intron-RdCVF-BGH (expression sequence set forth in SEQ ID NO: 99); C: pAV-p5-intron-CYP4V2-P2A-RdCVF-BGH (expression sequence set forth in SEQ ID NO: 113); and D: pAV-p5-intron-EGFP-BGH.
(2) The same amounts of iPSC-differentiated RPE cells of BCD patient (genotype: CYP4V2: c.802-8_810del17bpinsGC homozygous mutation) were infected by viruses A, B, C, and D (MOI = 1×10⁴) as well as virus A (1/2 MOI) + virus B (1/2 MOI) (hereinafter referred to as group E) (the production process for RPE cells refers to Example 11). The BCD patients were admitted to the Department of Ophthalmology, Peking University Third Hospital from November 2018 to December 2018. The Ethics Committee of Peking University Third Hospital approved all aspects of this study, and the informed consents for sample collection were obtained from the subjects (or their legal guardians). The urine samples of patients were handled according to the procedure of Example 11 within 1 hour after collection.
(3) After 72 hours, the lipid deposition was observed under a microscope.
(4) The cells were harvested and the cell medium supernatants (sup) were collected. The cells were washed with PBS and then lysed with RIPA lysis buffer (Beijing Applygen C1053-100) to obtain the cell lysate (lys), and the protein expression level was detected by Western blot. The following antibodies were used: anti-CYP4V2 (Atlas, HPA029122), anti-β-actin (Abclonal, AC026), goat-anti-rabbit (Abclonal, AS014), TXNL6 Antibody (H00115861-D01P).

The protein expression results were shown in Fig. 11A. CYP4V2 or RdCVF was not expressed in the iPSC-differentiated RPE cells of BCD patient in the blank control group M (Mock) without virus infection; CYP4V2 or RdCVF was not expressed while EGFP was expressed, in the iPSC-differentiated RPE cells of BCD patient in the virus-infected group D; the expression of CYP4V2 can be detected in the lysate of iPSC-differentiated RPE cells of BCD patient in the virus-infected group A; the expression of RdCVF can be detected in both the supernatant and the cell lysate of iPSC-differentiated RPE cells of BCD patient in the virus-infected group B; and the expression of CYP4V2 can be detected in the lysate and the expression of RdCVF can be detected in both the cell supernatant and the cell lysate, for the iPSC-differentiated RPE cells of BCD patient in virus-infected groups C and E.

The observation results for lipid deposition were shown in Fig. 11B. Compared with group M, the lipid depositions in groups A, C, and E were improved. Group D was not included for comparison due to the mutual infection of fluorescences from EGFP expression and lipid staining.

### Example 13 Selection of promoter in virus-transfected BCD mice

(1) The virus packaging followed the procedure of Example 7: A: pAV-p5-intron-CYP4V2-BGH (expression sequence set forth in SEQ ID NO: 93); B: pAV-OPEFS-CYP4V2-BGH (expression sequence set forth in SEQ ID NO: 90). Promoter A: p5-intron; Promoter B: OPEFS. Through the injection into the subretinal spaces of 1-month-old BCD mice (purchased from Biocytogen Pharmaceuticals (Beijing) Co., Ltd., Cyp4v3-/-), two viruses with promoters were injected to 10 mice, respectively, wherein the bilateral eyeballs were injected with the therapeutic vector (pAV-p5-intron-CYP4V2-BGH (expression sequence set forth in SEQ ID NO: 93)/pAV-OPEFS-CYP4V2-BGH (expression sequence set forth in SEQ ID NO: 90)), and the observation was performed after 3 months.
   Process for injection into the subretinal space:
   1) The experiment materials were prepared, the mice's pupils were dilated with 1% atropine, and then the mice were anesthetized by intraperitoneal injection of 80 mg/kg ketamine + 8 mg/kg xylazine.
   2) After the anesthesia, the pupils were dilated with 1% atropine again. Then the mice were placed in front of the animal experiment platform of the ophthalmic surgery microscope (Topcon, OMS800), and 0.5% proparacaine was dropped on the eyeballs of mice for local anesthesia.
   3) The fluorescein sodium stock solution was added to the virus at a concentration of fluorescein sodium : virus = 100:1, and mixed with a pipette.
   4) A minipore was pricked by insulin needle in advance in the ciliary pars plana of the mouse eyeball, through which a microsyringe needle passes to enter the vitreous chamber of the mouse eyeball. At this time, an appropriate amount of 2% hydroxymethyl cellulose was dropped on the mouse eyeball such that the mouse fundus can be seen under the microscope. Then the needle was inserted into the contralateral periphery retina while keeping off the lens. The viruses with sodium fluorescein were slowly pushed-in, with an injection volume of 1 µl in each eye and a virus concentration of 1×10⁹. The fluorescein sodium served as the indicator for judging whether it was injected into the subretinal space.
      After the operation, the surface of the eyeball was washed with normal saline and the mouse was placed in a cage to wait for waking up.
(2) Observation of the fundus crystalline deposition by *in vivo* fundus photography.
   1) For the blank control (KO) mice and the mice injected with pAV-p5-intron-CYP4V2-BGH (promoter A; expression sequence set forth in SEQ ID NO: 93) and pAV-OPEFS-CYP4V2-BGH (promoter B; expression sequence set forth in SEQ ID NO: 90), the pupils in the eyes of mice were dilated with 1% atropine, and then the mice were anesthetized by intraperitoneal injection of 80 mg/kg ketamine + 8 mg/kg xylazine.
   2) The anesthetized mice were held flat on the experiment platform of Micro III small animal retinal imaging system (Phoenix Research Laboratory, Micro III), and an appropriate amount of 2% hydroxymethyl cellulose was dropped on the mouse eyeball to improve the contact effect between the lens and the cornea.
   3) The positions of the eyes in mice were adjusted by lifting and rotating the experiment platform, and the focal length and light intensity were adjusted to obtain the fundus images of mice, which were taken by the Micro III software.
   4) After the photography was completed, the quantitative analysis on crystalline deposition was performed using GraphPad Prism software.

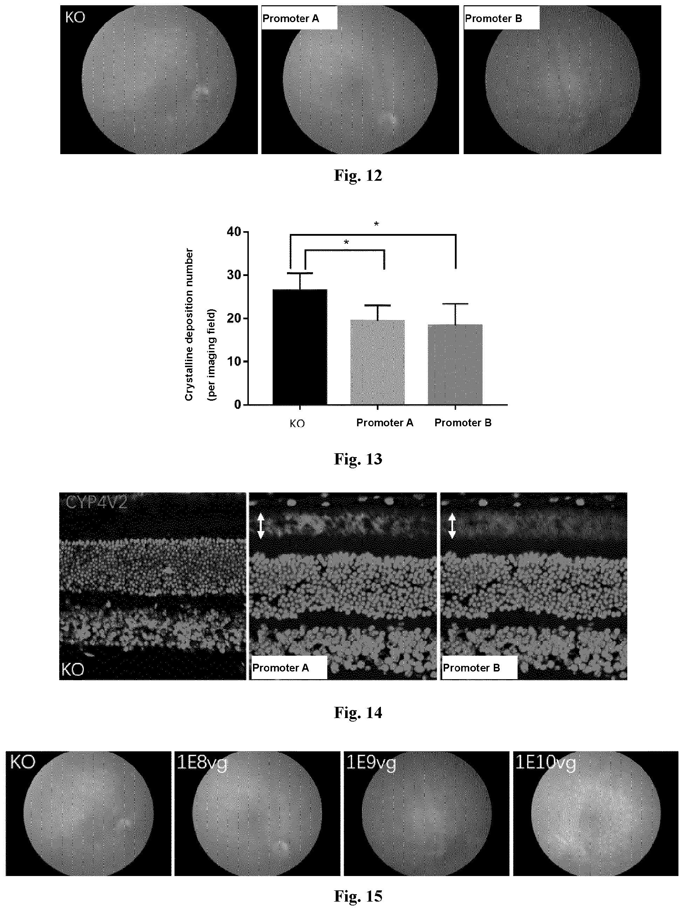

      Fig. 12 showed the fundus photography results. Fig. 13 showed the statistical result for the number of fundus crystals in mice after the injections into mice with viruses comprising different promoters. Figs. 12 and 13 showed that the fundus crystalline deposition was improved after injections of viruses containing different promoters.
(3) Morphological observation of retinal tissue:
   1) Fixation and dehydration: the mice were sacrificed by cervical dislocation to remove the eyeballs. The mouse eyeball was placed in a 1.5 ml EP tube, and 1 ml of 4% paraformaldehyde was added at 4°C overnight. Then the eyeball was transferred into a 1.5 ml EP tube containing 1 ml of 30% sucrose solution for dehydration, until the eyeball sank to the bottom.
   2) The mouse eyeball was placed into a 1.5 ml EP tube containing optimal cutting temperature compound (OCT). The mouse eye was positioned by tweezer to look straight ahead. The EP tube was capped and placed in liquid nitrogen. After being completely frozen, the frozen sections were obtained, with a section thickness of 7 µm. After fixation in acetone at 4°C for 10 min, they were stored at -80 °C.
   3) The sections were taken out, and after returning to room temperature, washed with PBS for 3 times (each for 5 min).
   4) The parts without tissues in the glass slide were wiped up. After dropping 40 µl of blocking solution (5% donkey serum) to each glass slide, the glass slide was blocked at room temperature for 1 h.
   5) The primary antibody was diluted with 5% donkey serum. About 40 µl of antibody working solution was dropped to completely cover the mouse eyeball tissues for incubating at 4°C overnight. The primary antibody used in this experiment was: CYP4V2 (1:50, purchased from Sigma).
   6) The glass slide was taken out and washed with PBS for 3 times (each for 5 min).
   7) The secondary antibody (purchased from Thermo Fisher Scientific) was diluted with PBS at a ratio of 1:800. About 40 µl of secondary antibody working solution was dropped to completely cover the mouse eyeball tissues for incubating at room temperature for 1 h. The primary antibody used in this experiment was: CYP4V2 (1:50, purchased from Sigma).
   8) The glass slide was washed with PBS for 3 times, and DAPI diluent (1:5000) was added for incubating at room temperature for 15 min.
   9) After dropping the anti-fluorescence-quenching mounting medium, the glass slide was covered with a coverslip, and stored at -20°C in dark.
   10) The Nikon A1 laser confocal microscope equipped with NIS-Elements C software was used for observation and imaging.

The immunofluorescence results in Fig. 14 showed that CYP4V2 was expressed in BCD mice injected with viruses comprising promoter A and injected with viruses comprising promoter B (double arrows indicate the range in the figure).

### Example 14 Dose selection in virus-infected BCD mice

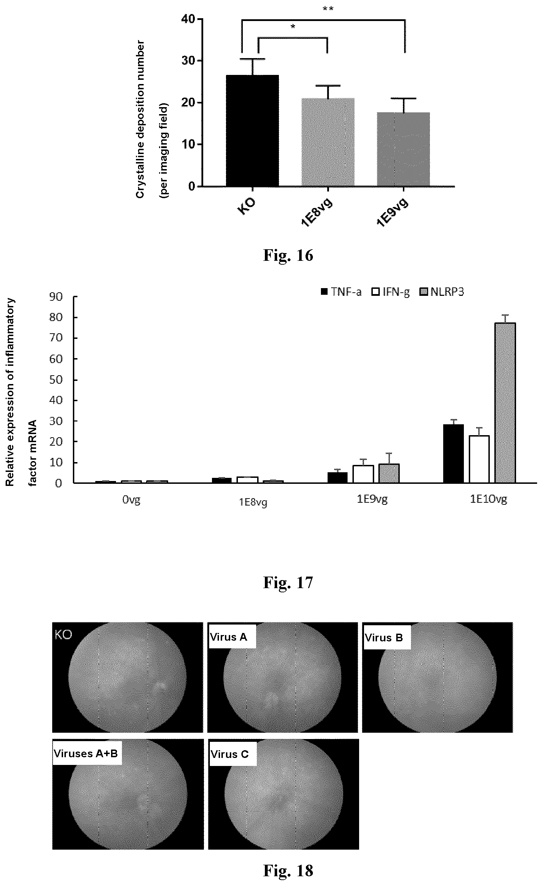

(1) The virus dose was verified according to the method of Example 13. Through the injection into the subretinal spaces of 1-month-old BCD mice (Cyp4v3-/-), the bilateral eyeballs were injected with the therapeutic vector pAV-OPEFS-CYP4V2-BGH (expression sequence set forth in SEQ ID NO: 90) for the observation after 3 months, wherein the viruses at 1E8 vg, 1E9 vg, and 1E10 vg were injected in each eye, respectively.
   The crystalline deposition was shown in the photographic results in Fig. 15. Fig. 16 showed the statistical result for the fundus crystal numbers under identical area in BCD mice injected with viruses at 1E8 vg and 1E9 vg and not injected with viruses. Compared with BCD mice not injected with viruses, BCD mice injected with viruses at 1E8 vg and 1E9 vg showed decreased numbers of crystal deposits, wherein the number of crystal deposits in BCD mice injected with viruses at 1E9 vg was significantly decreased. BCD mice injected with viruses at 1E10 vg showed no significant improvement in crystalline deposition, with a severe damage.
(2) The RPE cell layer was peeled off for RNA extraction, and the expression levels of inflammatory factors TNF-α, IFN-γ, and NLRP3 were detected. The RNA extraction and reverse transcription were performed in accordance with the procedure in step (4) of Example 11, and RT-qPCR was performed in accordance with the procedure in step III of Example 10. The qPCR primer sequences were set forth in SEQ ID NOs: 146-153.

Fig. 17 showed that the injection of viruses at 1E10 vg results in increased expressions of inflammatory factors TNF-α, IFN-γ, and NLRP3, indicating that the viral dose at 1E10 vg had toxic and side effects in BCD mice, and the dose at 1E9 vg was superior to that at 1E8 vg. Thus, a viral dose at 1E10 vg or less was chosen for subsequent experiments.

### Example 15 Effects in BCD mice transfected by various viruses

### I. Effects in BCD mice infected by viruses with p5-intron promoter

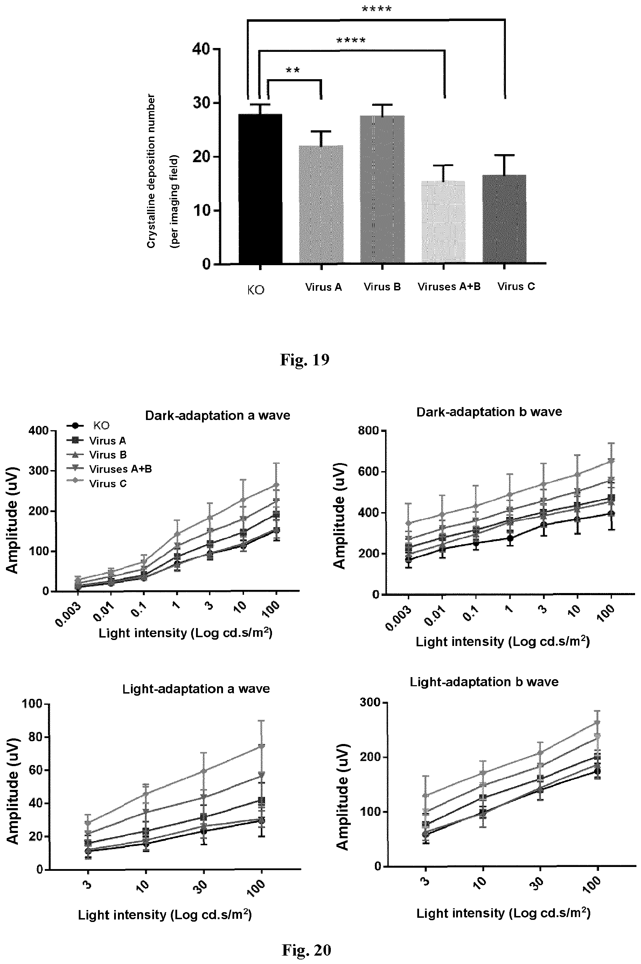

(1) The virus packaging followed the procedure of Example 7: A: pAV-p5-intron-CYP4V2-BGH (expression sequence set forth in SEQ ID NO: 93); B: pAV-p5-intron-RdCVF-BGH (expression sequence set forth in SEQ ID NO: 99); C: pAV-p5-intron-CYP4V2-P2A-RdCVF-BGH (expression sequence set forth in SEQ ID NO: 113).
(2) The 1-month-old BCD mice (Cyp4v3-/-) were divided into five groups: group KO, group A, group B, group A+B, and group C, using viruses A, B, and C at 1E9 vg as well as virus A + virus B (A and B at 5E8 vg, respectively), and group KO without treatment as the blank control. The injection into the subretinal space was performed in accordance with the procedure in Example 13.
(3) In accordance with the procedure of Example 13, the crystalline deposition was observed by *in vivo* fundus photography. Fig. 18 showed the results of fundus photography for the crystalline deposition in BCD mice after the treatments with various viruses through injection into the subretinal space. Fig. 19 showed the statistical result for the number of fundus crystals in BCD mice after the treatments with various viruses through injection into the subretinal space (n=5). The results showed that the numbers of crystals in mice in the virus group A+B and the virus group C decreased more significantly. There was no significant improvement in the fundus crystal for the mice in the virus group B.
*(4) In vivo* retinal function test: electroretinogram (ERG)
   1) Dark adaptation in mice: the mice were subjected to the dark adaptation for at least 16 hours, after which all the operations were performed under dark red light.
   2) Anesthesia in mice: the anesthesia was performed by intraperitoneal injection of 80 mg/kg ketamine + 8 mg/kg xylazine.
   3) After the anesthesia was completed, the pupils in the eyes of mice were dilated with 1% atropine under the illumination of dark red light. The mice were fixed with adhesive tape in front of the animal experiment platform of the visual electrophysiology instrument Espion E2, and the eyes were consistent and fully exposed. The ground electrode needle was inserted into the root of the mouse tail, and the reference electrode needle was inserted into the mouse jaw. Two gold ring recording electrodes were clamped on the electrode holder of the animal experiment platform, and their angles were adjusted so that they slightly touch the top end in the center of the left and right corneas respectively. An appropriate amount of 2% hydroxymethyl cellulose was dropped to improve the contact effect between the gold ring electrode and the cornea.
   4) The information about mouse number and age was entered in the Espion E2 computer system and then the program was run. The dark-adaptation flash intensity was 0.003, 0.01, 0.1, 1, 3, 10, and 100 cd·s/m² respectively (the background light intensity was 0 cd·s/m², the stimulation interval was 15 s, and the average of three ERG signals was recorded); and the light-adaptation flash intensity was 3, 10, 30, and 100 cd·s/m² respectively (the light-adaptation time was 5 min, the background light intensity was 30 cd·s/m², the stimulation interval was 15 s, and the average of five ERG signals was recorded). After the program was completed, the running results were automatically saved, and GraphPad Prism was used for the result statistics.
   Fig. 20 showed the ERG record results for BCD mice after the treatments with various viruses through injection into the subretinal space. The results showed that the ERG amplitude for the mice in the virus group B did not change significantly. The ERG amplitude was increased (p<0.05) in mice in the virus groups A, A+B, and C, and that in the virus group A+B was superior to that in the virus group A (p<0.05).
(5) The expressions of CYP4V2 and RdCVF in BCD mice were detected in accordance with the procedure in step (3) of Example 13. RdCVF (1:50, H00115861-D01P).

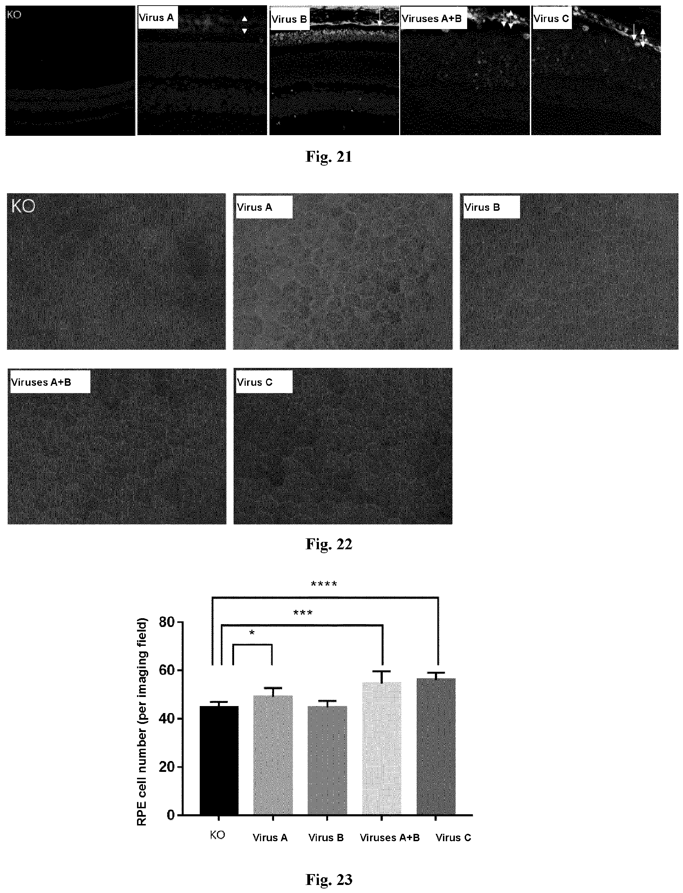

   Fig. 21 showed the immunofluorescence staining images for BCD mice after the treatments with various viruses through injection into the subretinal space. The results showed that CYP4V2 was expressed in the mice in virus group A (double arrows indicate the range), RdCVF was expressed in the mice in virus group B (single arrows indicate the range), and the expressions of CYP4V2 and RdCVF can be detected in both the virus group A+B and the virus group C.
(6) Detection of the number and morphology of RPE cells by mouse eyeball RPE stretched preparation and staining
   1) The pre-chilled PBS was added to a 1.5 ml EP tube. After the mice were sacrificed, the eyeballs of the mice were removed and immersed in PBS for 15 min.
   2) The mouse eyeball was transferred to a 1.5 ml EP tube containing 1 ml of 4% paraformaldehyde, and fixed for 1 h.
   3) The anterior segment of the mouse eyeball was removed under a stereomicroscope (Olympus, SZ61-SET), the neural retinal layer was separated from the RPE layer, and the RPE layer was cut into 4 flaps.
   4) The RPE stretched preparation was washed in the pre-chilled PBS for 3 times (each for 5 min).
   5) The RPE stretched preparation was permeabilized in 0.1% Triton for 20 min, then washed with PBS for 3 times (each for 5 min).
   6) The RPE stretched preparation was placed into one well of a 96-well plate, and incubated at room temperature for 1 h after the Phalloidin working solution diluted with PBS at a ratio of 1:200 was added.
   7) The RPE stretched preparation was washed with PBS for 3 times (each for 5 min), flatten on a glass slide which was then mounted by a coverslip after dropping a small amount of mounting medium, and observed under Nikon fluorescence microscope.

As shown in Fig. 22, the results of Phalloidin-labeled F-actin staining showed that compared with BCD mice not injected with viruses, the RPE cells had more intact hexagonal morphology and dense arrangements in BCD mice injected with viruses comprising promoter A and in BCD mice injected with viruses comprising promoter B.

As shown in Fig. 22, the results of Phalloidin-labeled F-actin staining showed that compared with group KO, group B had no obvious change; compared with other three groups, the RPE cells had more intact hexagonal morphology and dense arrangements in the virus group A+B and the virus group C. As shown in Fig. 23, the statistical result by counting the number of RPE cells in the same area showed that the numbers of RPE cells in the same area in the virus group A+B and the virus group C were larger than that in the virus group A (n=5).

In comprehensive consideration of the observation results of crystalline deposition, electroretinogram (ERG), and RPE cell number and morphology, it can be known that: the expression of RdCVF alone (group B) did not improve the crystalline deposition, electroretinogram (ERG), and RPE cell number and morphology in BCD mice; and compared with the improvements in crystalline deposition, electroretinogram (ERG), and RPE cell number and morphology by expressing CYP4V2 alone in BCD mice, such improvements were significantly enhanced by the co-expression of RdCVF and CYP4V2 (group A+B and group C), indicating that the co-expression of CYP4V2 and RdCVF had a significant synergistic effect over the expression of CYP4V2 or RdCVF alone.

### II. ERG effects in BCD mice infected by viruses with no intron following the promoter

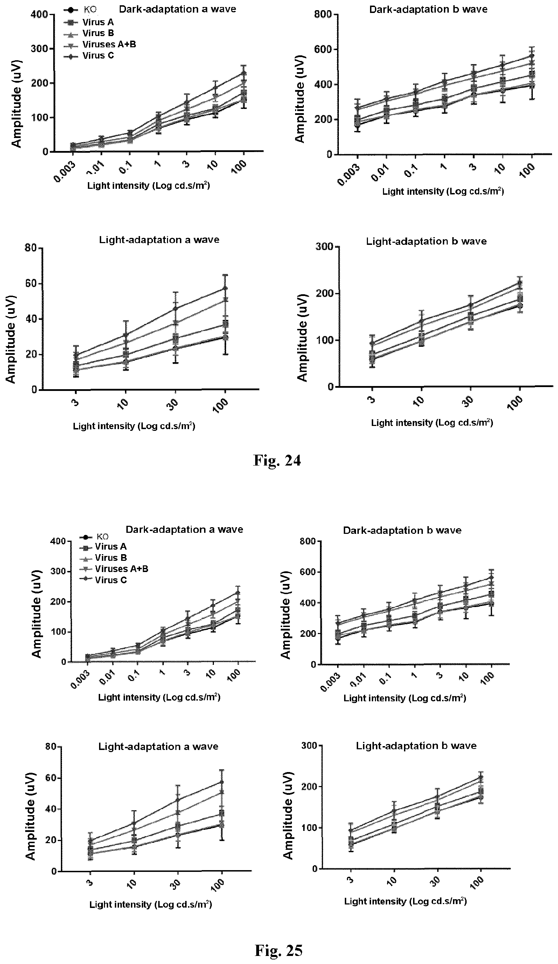

(1) The virus packaging followed the procedure of Example 7: A: pAV-p5-CYP4V2-BGH (expression sequence set forth in SEQ ID NO: 91); B: pAV-p5-RdCVF-BGH (expression sequence set forth in SEQ ID NO: 97); C: pAV-p5-CYP4V2-P2A-RdCVF-BGH (expression sequence set forth in SEQ ID NO: 107).
(2) The 1-month-old BCD mice (Cyp4v3-/-) were divided into five groups: group KO, group A, group B, group A+B, and group C, using viruses A, B, and C at 1E9 vg as well as virus A + virus B (A and B at 5E8 vg respectively), and group KO with no treatment as the blank control. The injection into the subretinal space was performed in accordance with the procedure in Example 13.
(3) In accordance with the procedure in step I (4) of this Example, the *in vivo* retinal function test (electroretinogram (ERG)) was performed. Fig. 24 showed the ERG record results for BCD mice after the treatments with the aforementioned various viruses through injection into the subretinal space. The results showed that the ERG amplitude for the mice in the virus group B did not change significantly. The ERG amplitude was increased (p<0.05) in mice in the virus groups A, A+B, and C, and that in the virus group A+B was superior to that in the virus group A (p<0.05).

### III. ERG Effects in BCD mice infected by viruses with p1 promoter

(1) The virus packaging followed the procedure of Example 7: A: pAV-p1-CYP4V2-BGH (expression sequence set forth in SEQ ID NO: 92); B: pAV-p1-RdCVF-BGH (expression sequence set forth in SEQ ID NO: 98); C: pAV-p1-CYP4V2-P2A-RdCVF-BGH (expression sequence set forth in SEQ ID NO: 108).
(2) The 1-month-old BCD mice (Cyp4v3-/-) were divided into five groups: group KO, group A, group B, group A+B, and group C, using viruses A, B, and C at 1E9 vg as well as virus A + virus B (A and B at 5E8 vg respectively), and group KO with no treatment as the blank control. The injection into the subretinal space was performed in accordance with the procedure in Example 13.
(3) In accordance with the procedure in step I (4) of this Example, the *in vivo* retinal function test (electroretinogram (ERG)) was performed. Fig. 25 showed the ERG record results for BCD mice after the treatments with the aforementioned various viruses through injection into the subretinal space. The results showed that the ERG amplitude for the mice in the virus group B did not change significantly. The ERG amplitude was increased (p<0.05) in mice in the virus groups A, A+B, and C, and that in the virus group A+B was superior to that in the virus group A (p<0.05).

### IV. ERG effects in BCD mice infected by viruses with WPRE-SV40 PolyA signal site

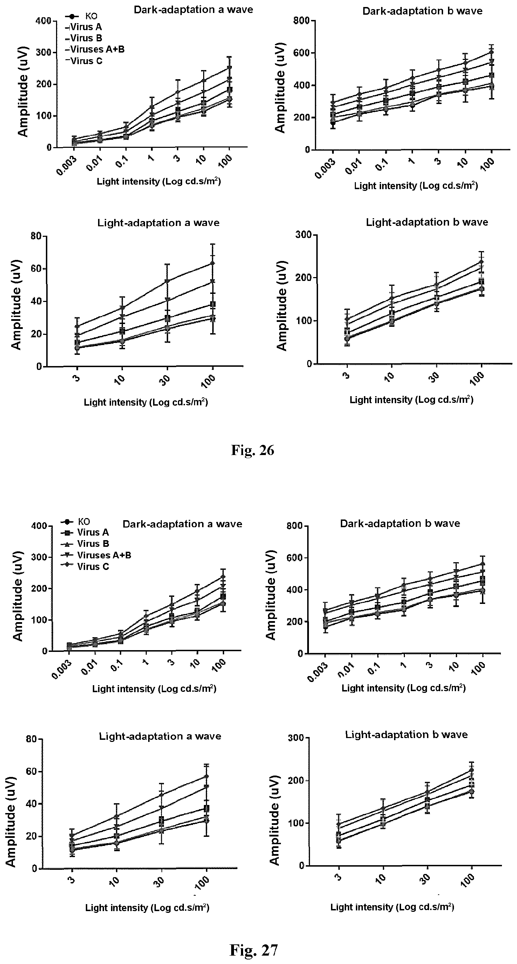

(1) The virus packaging followed the procedure of Example 7: A: pAV-p5-intron-CYP4V2-WPRE-SV40 (expression sequence set forth in SEQ ID NO: 94); B: pAV-p5-intron-RdCVF-WPRE-SV40 (expression sequence set forth in SEQ ID NO: 100); C: pAV-p5-intron-CYP4V2-P2A-RdCVF-WPRE-SV40 (expression sequence set forth in SEQ ID NO: 114).
(2) The 1-month-old BCD mice (Cyp4v3-/-) were divided into five groups: group KO, group A, group B, group A+B, and group C, using viruses A, B, and C at 1E9 vg as well as virus A + virus B (A and B at 5E8 vg respectively), and group KO with no treatment as the blank control. The injection into the subretinal space was performed in accordance with the procedure in Example 13.
(3) In accordance with the procedure in step I (4) of this example, the *in vivo* retinal function test (electroretinogram (ERG)) was performed. Fig. 26 showed the ERG record results for BCD mice after the treatments with the aforementioned various viruses through injection into the subretinal space. The results showed that the ERG amplitude for the mice in the virus group B did not change significantly. The ERG amplitude was increased (p<0.05) in mice in the virus groups A, A+B, and C, and that in the virus group A+B was superior to that in the virus group A (p<0.05).

### V. Effects in BCD mice infected by viruses without signal site

(1) The virus packaging followed the procedure of Example 7: A: pAV-p5-CYP4V2 (set forth in SEQ ID NO: 117); B: pAV-p5-RdCVF (set forth in SEQ ID NO: 120); C: pAV-p5-CYP4V2-P2A-RdCV (set forth in SEQ ID NO: 122).
(2) The 1-month-old BCD mice (Cyp4v3-/-) were divided into five groups: group KO, group A, group B, group A+B, and group C, using viruses A, B, and C at 1E9 vg as well as virus A + virus B (A and B at 5E8 vg respectively), and group KO with no treatment as the blank control. The injection into the subretinal space was performed in accordance with the procedure in Example 13.
(3) In accordance with the procedure in step I (4) of this example, the *in vivo* retinal function test (electroretinogram (ERG)) was performed.

Fig. 27 showed the ERG record results for BCD mice after the treatments with the aforementioned various viruses through injection into the subretinal space. The results showed that compared with the control, the ERG amplitude for the mice in the virus group B did not change significantly. The ERG amplitude was increased in mice in the virus groups A, A+B, and C, and that in the virus group A+B was superior to that in the virus group A (p<0.05).

The foregoing detailed descriptions are provided by way of explanation and illustration, and are not intended to limit the scope of the appended claims. Various modifications to the embodiments presently listed in the present application will be apparent to those of ordinary skill in the art and remained within the scope of the appended claims and equivalents thereof.

## Claims

1. A vector sequentially comprising, in 5' to 3' direction: a promoter, a polynucleotide encoding CYP4V2 and a PolyA signal site, wherein the promoter is operably linked to the polynucleotide encoding CYP4V2, and the promoter is CAG promoter.

2. The vector according to claim 1, wherein the CAG promoter comprises a nucleotide sequence set forth in SEQ ID NO: 4.

3. The vector according to claim 1 or 2, wherein the CYP4V2 comprises an amino acid sequence set forth in any of SEQ ID NOs: 76-82.

4. The vector according to any of claims 1 to 3, wherein the polynucleotide encoding CYP4V2 comprises a nucleic acid sequence set forth in SEQ ID NO: 62 or a nucleic acid sequence having at least 95% identity to the nucleic acid sequence set forth in SEQ ID NO: 62.

5. The vector according to any of claims 1 to 4, wherein the PolyA signal site comprises a nucleic acid sequence set forth in any of SEQ ID NOs: 17-21.

6. The vector according to any of claims 1 to 5, wherein the vector is AAV2/2, AAV2/5, AAV2/8, or AAV2/9.

7. The vector according to claim 6, wherein the vector is AAV2/8.

8. A cell comprising a vector according to any of claims 1 to 7.

9. A pharmaceutical composition comprising a vector according to any of claims 1 to 7 and/or a cell according to claim 8.

10. The vector according to any of claims 1 to 7 and/or the cell according to claim 8 for use in a method for treating, alleviating, and/or preventing a disease or disorder associated with retinal pigment epithelium (RPE) atrophy.

11. The vector according to any of claims 1 to 7 and/or the cell according to claim 8 for use according to claim 10, wherein the disease or disorder is Bietti's crystalline dystrophy.

12. The vector according to any of claims 1 to 7 and/or the cell according to claim 8 for use according to claim 10 or 11, wherein the subject is human.
